# EUROPÄISCHE PATENTSCHRIFT

(11) **EP 2 593 528 B1**
(45) Veröffentlichungstag und Bekanntmachung des Hinweises auf die Patenterteilung: **29.10.2014**
(21) Anmeldenummer: 11728781.3
(22) Anmeldetag: 24.06.2011
(51) Int. Cl.: C09K 19/34, C09K 19/42, C09K 19/54, C09K 19/04, C07D 333/08, C07D 333/12, C07D 333/48

(54) **FLÜSSIGKRISTALLINES MEDIUM MIT STABILISATOR**
LIQUID-CRYSTALLINE MEDIUM WITH STABILIZER
MILIEU CRISTALLIN LIQUIDE PRÉSENTANT UN AGENT STABILISATEUR

(30) Priorität: 14.07.2010 DE 102010027099
(43) Veröffentlichungstag der Anmeldung: 22.05.2013
(73) Patentinhaber: Merck Patent GmbH, 64293 Darmstadt (DE)
(72) Erfinder: HIRSCHMANN, Harald, 64291 Darmstadt (DE); HOCK, Christian, 63814 Mainaschaff (DE); LIETZAU, Lars, 64295 Darmstadt (DE); JAEHRLING, Kai, 64686 Lautertal (DE); JANSEN, Axel, 64293 Darmstadt (DE); KLASEN-MEMMER, Melanie, 67259 Heuchelheim (DE)
(86) Internationale Anmeldenummer: PCT/EP2011/003131
(87) Internationale Veröffentlichungsnummer: WO 2012/007096

(56) Entgegenhaltungen:
- WO-A1-2008/084109
- DE-A1-102009 015 554
- SEED A J ET AL: "NOVEL, HIGHLY POLARIZABLE THIOPHENE DERIVATIVES FOR USE IN NONLINEAR OPTICAL APPLICATIONS", LIQUID CRYSTALS: AN INTERNATIONAL JOURNAL OF SCIENCE AND TECHNOLOGY, TAYLOR & FRANCIS, GB, Bd. 30, Nr. 9, 1. September 2003 (2003-09-01), Seiten 1089-1107, XP001169724, ISSN: 0267-8292, DOI: 10.1080/0267829031000154363
- MATHARU A S ET AL: "LATERALLY FLUORINATED LIQUID CRYSTALS CONTAINING THE 2,2'-BITHIOPHENE MOIETY", LIQUID CRYSTALS: AN INTERNATIONAL JOURNAL OF SCIENCE AND TECHNOLOGY, TAYLOR & FRANCIS, GB, Bd. 34, Nr. 4, 1. April 2007 (2007-04-01), Seiten 489-506, XP001540578, ISSN: 0267-8292, DOI: 10.1080/02678290601176559 in der Anmeldung erwähnt

## Beschreibung

Die vorliegende Erfindung betrifft flüssigkristalline Medien (FK-Medien) mit Thiophenderivaten, die durch Dithiophene und Thiophenoxide stabilisiert werden und Flüssigkristallanzeigen (FK-Anzeigen) enthaltend diese FK-Medien. Ein weiterer Gegenstand sind Thiophenoxid-Verbindungen, die auch als Stabilisatoren in diesen FK-Medien eingesetzt werden können.

Flüssige Kristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur. Daneben gibt es auch Zellen, die mit einem elektrischen Feld parallel zur Substrat- und Flüssigkristallebene arbeiten, wie beispielsweise die IPS-Zellen ("in-plane switching").

Das Prinzip der elektrisch kontrollierten Doppelbrechung, der ECB-Effekt (electrically controlled birefringence) oder auch DAP-Effekt (Deformation aufgerichteter Phasen) wurde erstmals 1971 beschrieben (M.F. Schieckel und K. Fahrenschon, "Deformation of nematic Liquid crystals with vertical orientation in electrical fields", Appl. Phys. Lett. 19 (1971), 3912). Es folgten Arbeiten von J.F. Kahn (Appl. Phys. Lett. 20 (1972), 1193) und G. Labrunie und J. Robert (J. Appl. Phys. 44 (1973), 4869).

Die Arbeiten von J. Robert und F. Clerc (SID 80 Digest Techn. Papers (1980), 30), J. Duchene (Displays 7 (1986), 3) und H. Schad (SID 82 Digest Techn. Papers (1982), 244) haben gezeigt, dass flüssigkristalline Phasen hohe Werte für das Verhältnis der elastischen Konstanten K₃/K₁, hohe Werte für die optische Anisotropie Δn und Werte für die dielektrische Anisotropie Δε≤ -0,5 aufweisen müssen, um für hochinformative Anzeigeelemente basierend auf dem ECB-Effekt eingesetzt werden zu können. Auf dem ECB-Effekt basierende elektrooptische Anzeigeelemente weisen eine homeotrope Randorientierung auf (VA-Technologie = Vertical Aligned). Auch bei Anzeigen, die den IPS- oder den FFS-Effekt verwenden, können dielektrisch negative Flüssigkristallmedien zum Einsatz kommen.

Vor allem die TN-, VA- und IPS- Zellen sind derzeit kommerziell interessante Einsatzgebiete für die erfindungsgemäßen Medien.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem möglichst breiten Bereich unterhalb und oberhalb Raumtemperatur eine geeignete Mesophase besitzen, beispielsweise für die oben genannten Zellen eine nematische Mesophase. Da Flüssigkristalle in der Regel als Mischungen mehrerer Komponenten zur Anwendung gelangen, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nicht-linearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand, guter UV- und Temperaturstabilität, und geringem Dampfdruck erwünscht.

Derartige Matrix-Flüssigkristallanzeigen sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die TFT-Anzeigen arbeiten üblicherweise als TN-Zellen mit gekreuzten Polarisatoren in Transmission und sind von hinten beleuchtet.

Der Begriff MFK-Anzeigen umfasst hier jedes Matrix-Display mit integrier ten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfernseher) oder für hochinformative Displays für Rechneranwendungen (Laptop) und im Automobil- oder Flugzeugbau. Neben Problemen hinsichtlich der Winketabhängigkeit des Kontrastes und der Schaltzeiten resultieren bei MFK-Anzeigen Schwierigkeiten bedingt durch nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten. Insbesondere bei low-volt-Mischungen war es bisher nicht möglich, sehr hohe spezifische Widerstände zu realisieren. Weiterhin ist es wichtig, dass der spezifische Widerstand eine möglichst geringe Zunahme bei steigender Temperatur sowie nach Temperatur- und/oder UV-Belastung zeigt. Die MFK-Anzeigen aus dem Stand der Technik genügen somit nicht den heutigen Anforderungen.

Für TV- und Videoanwendungen werden MFK-Anzeigen mit geringen Schaltzeiten benötigt. Solche geringen Schaltzeiten lassen sich besonders dann realisieren, wenn Flüssigkristallmedien mit geringen Werten für die Viskosität, insbesondere der Rotationsviskosität γ1 verwendet werden. Verdünnende Zusätze verringern jedoch in der Regel den Klärpunkt und damit den Arbeitstemperaturbereich des Mediums.

Es besteht somit immer noch ein großer Bedarf nach MFK-Anzeigen mit sehr hohem spezifischen Widerstand bei gleichzeitig großem Arbeitstemperaturbereich, kurzen Schaltzeiten auch bei tiefen Temperaturen und niedriger Schwellenspannung, die diese Nachteile nicht oder nur in geringerem Maße zeigen.

Bei TN-(Schadt-Helfrich)-Zellen sind Medien erwünscht, die folgende Vorteile in den Zellen ermöglichen:
- erweiterter nematischer Phasenbereich (insbesondere zu tiefen Temperaturen)
- Schaltbarkeit bei extrem tiefen Temperaturen (out-door-use, Automobil, Avionik)
- erhöhte Beständigkeit gegenüber UV-Strahlung (längere Lebensdauer),
- kleine Schwellenspannung

Mit den aus dem Stand der Technik zur Verfügung stehenden Medien ist es nicht möglich, diese Vorteile unter gleichzeitigem Erhalt der übrigen Parameter zu realisieren.

Der Erfindung liegt als eine Aufgabe zugrunde, Medien insbesondere für derartige TN-, VA- oder IPS-Anzeigen bereitzustellen, welche die oben angegebenen gewünschten Eigenschaften besitzen und die oben angegebenen Nachteile nicht oder nur in geringerem Maße zeigen. Insbesondere sollten die FK-Medien schnelle Schaltzeiten und niedrige Rotationsviskositäten bei gleichzeitig hoher dielektrische Anisotropie und hoher Doppelbrechung aufweisen. Darüber hinaus sollten die FK-Medien einen hohen Klärpunkt, einen breiten nematischen Phasenbereich und eine niedrige Schwellenspannung aufweisen.

Es wurde nun gefunden, dass diese Aufgabe gelöst werden kann, wenn man (Di-)Thiophenderivate in FK-Medien, insbesondere in FK-Medien mit aktiver Adressierung, und in TN-, VA- und IPS-Anzeigen, verwendet. Die unten angegebene Kombination von (Di-)Thiophenderivaten führen zu FK-Medien mit den oben angegebenen gewünschten Eigenschaften.

Gegenstand der Erfindung ist ein stabilisiertes FK-Medium, das Thiophenderivate und geeignete Stabilisatoren umfasst.

Thiophenhaltige FK-Medien sind z.B. aus den Druckschriften DP0467260 A2, GB 2229179 A, DE 102005 01554 A1, WO 2008/084109 A1 A.J. Seed et al., Liquid Crystals 2003, 30, 1089-1107 und A. S. Matharu et al., Liquid Crystals 2007, 34, 489-506 bekannt.

Die EP 0 467 260 A2 offenbart Verbindungen mit einer Thiophen-2,5-diyleinheit, die direkt mit einer 2-und/oder 3-substiuierten Phenylen-1,4-diyleinheit verknüpft ist. Die der EP 0 467 260 A2 zugrundeliegende Aufgabe war jedoch die Entwicklung neuer Materialien für die Anwendung in ferroelektrischen FK-Anzeigen. Deshalb sind auch insbesondere ferroelektrische FK-Medien und ferroelektrische FK-Substanzen, sowie allgemein Verbindungen, die besonders für den Einsatz in ferroelektrischen FK-Anzeigen geeignete Eigenschaften aufweisen, Gegenstand der EP 0 467 260 A2.

Ferroelektrische FK-Anzeigen sind heutzutage jedoch nur noch von untergeordneter Bedeutung. Für moderne Displayanwendungen werden fast ausschließlich nematische Flüssigkristallmedien verwendet. Die in der EP 0 467 260 A2 beschriebenen Verbindungen sind für solche modernen Displayanwendungen jedoch ungeeignet, da fast ausschließlich smektische Flüssigkristalle beschrieben werden.

In A. S. Matharu et al., Liquid Crystals 2007, 34, 489-506 werden lateral fluorierte Flüssigkristalle mit 2,2'-Bithiopheneinheiten beschrieben, darunter auch Verbindungen der folgenden Formeln 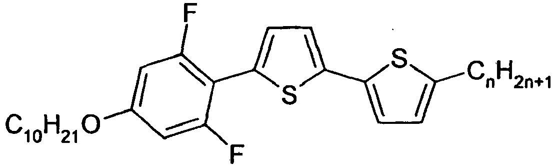 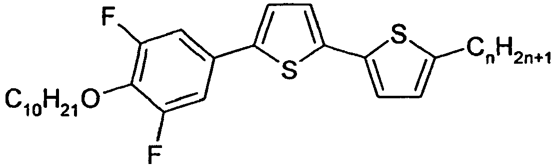 wobei Substanzen mit n = 6-10 synthetisiert und untersucht wurden. Die Substanzen weisen aufgrund der Länge der Alkyl- bzw. Alkoxyseitenketten und der zentralen Thiopheneinheit schlechte Phaseneigenschaften auf. So werden bei niedrigen Klärpunkten lediglich schmale monotrope smektisch A-Phasen ausgebildet (z.B. für n = 6: K 30,1 SmA (24,8) I). FK-Medien mit diesen Verbindungen wurden nicht untersucht.

In der EP 0 499 252 A1 und einer Reihe ähnlicher Veröffentlichungen EP 0 459 406 A1, EP 0 476 567 A1, EP 0 500 072 A1, JP 06-025668 A werden Thiophenderivate der folgenden Formel 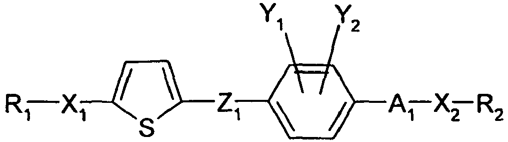 beschrieben, wobei Z₁ COO, CH₂O oder CH=C(CN) bedeutet, und Y₁ und Y₂ neben anderen Bedeutungen auch F bedeuten können. Als konkrete Beispiele werden folgende Verbindungen beschrieben (siehe z.B. EP 0 499 252 A1, Verbindungen I-110 auf Seite 32 und I-130 auf Seite 35):

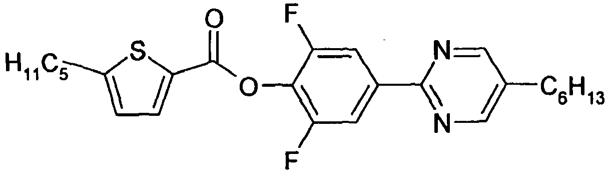

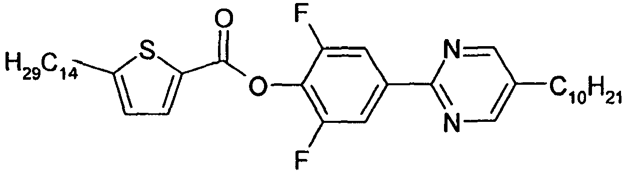

Auch diese Dokumente zielen auf die Entwicklung neuer Materialien für die Anwendung in ferroelektrischen FK-Anzeigen ab. Damit weisen auch die darin beschriebenen Verbindungen die oben erwähnten Nachteile (z.B. smektische Phasen, schlechte VHR durch Estergruppen) in Bezug auf Ihre Verwendung in modernen, nematischen Flüssigkristallmedien auf.

In der GB 2 229 179 A werden Alkylthiophene vom Typ der Formel 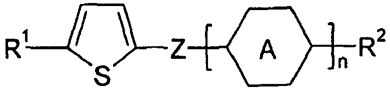 beschrieben, wobei R¹, R² unter Anderem Alkylreste mit bis zu 16 Kohlenstoffatomen oder CN oder F bedeuten können, Z eine COO-, OCO-, CH₂CH₂- oder CH₂O-Brück bedeutet, A unter Anderem ein fluorierter Phenylrest sein kann, und n 1 oder 2 bedeuten kann. Die Aufgabe der dort beschriebenen Erfindung war es jedoch, Verbindungen mit moderater bzw. niedriger Doppelbrechung zu erhalten. Dazu wurde die Konjugation zwischen der Thiopheneinheit und aromatischen Einheiten A bewusst durch Einführung von nicht zur Konjugation befähigten Brückengliedern unterbrochen. Für moderne TN-TFT-Displayanwendungen werden allerdings bevorzugt Komponenten benötigt, die eine höhere Doppelbrechung mit einem guten Rotationsviskosität zu Klärpunktverhältnis vereinen.

In der Druckschrift Brettle et al. New J. Chem 1994, 18, 643-648 werden Thiophenoxide mit einem angegliederten Benzolring auf ihre mesogenen Eigenschaften hin untersucht.

Wie zuvor schon erläutert wurde, wirken sich weiterhin die hier besonders bevorzugten Estergruppen negativ auf die 'Reliability' von Flüssigkristallen aus. Beispiele für Komponenten mit Ethylenbrücken bzw. CH₂O-Gruppen zwischen einer Thiopheneinheit und einem weiteren aromatischen Ringelement werden nicht offenbart. Weiterhin wird nicht auf das Fluorierungsmuster eines mit der Thiopheneinheit verbrückten aromatischen Ringsystems eingegangen.

Es war deshalb für den Fachmann aufgrund des Standes der Technik nicht zu erwarten, dass die Verwendung von erfindungsgemäßen Thiophenderivaten in achiralen nematischen FK-Medien mit einer inhärent unverdrillten Phase, insbesondere in FK-Medien mit positiver dielektischer Anisotropie, und in TN-, VA- und IPS-Anzeigen, zu einer Verbesserung der Eigenschaften, insbesondere zu schnellen Schaltzeiten und niedrigen Rotationsviskositäten bei gleichzeitig hoher dielektrischer Anisotropie, hoher Doppelbrechung und hohem spezifischen Widerstand, führen kann.

Gegenstand der vorliegenden Erfindung ist somit ein FK-Medium enthaltend Verbindungen der Formel I 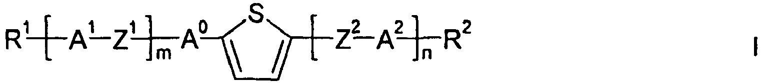 worin die einzelnen Reste folgende Bedeutung besitzen:
- R¹ und R²: H, F, Cl, Br, -CN, -SCN, -NCS, SF₅ oder geradkettiges oder verzweigtes Alkyl mit 1 bis 12 C-Atomen, worin auch eine oder mehrere nicht benachbarte CH₂-Gruppen jeweils unabhängig voneinander durch -CH=CH-, -C≡C-, -O-, -CO-, -CO-O-, -O-CO-, -O-CO-O- so ersetzt sein können, dass O-Atome nicht direkt miteinander verknüpft sind, und worin auch ein oder mehrere H-Atome durch F, Cl oder Br ersetzt sein können, oder P-Sp-,
- A⁰, A¹, A²: jeweils unabhängig voneinander Phenylen-1,4-diyl, worin auch eine oder zwei CH-Gruppen durch N ersetzt sein können und ein oder mehrere H-Atome durch Halogen, CN, CH₃, CHF₂, CH₂F, OCH₃, OCHF₂ oder OCF₃ ersetzt sein können, Cyclohexan-1,4-diyl, worin auch eine oder zwei nicht benachbarte CH₂ -Gruppen unabhängig voneinander durch O und/oder S ersetzt sein können, und ein oder mehrere H-Atome durch F ersetzt sein können, Cyclohexen-1,4-diyl, Bicyclo[1.1.1]pentan-1,3-diyl, Bicyclo[2.2.2]octan-1,4-diyl, Spiro[3.3]heptan-2,6-diyl, Tetrahydropyran-2,5-diyl, oder 1,3-Dioxan-2,5-diyl,
- Z¹ und Z²: jeweils unabhängig voneinander -CF₂O- -OCF₂-, -CH₂O-, -OCH₂₋, -CO-O-, -O-CO-, -C₂H₄- -C₂F₄-, -CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- oder eine Einfachbindung,
- m und n: jeweils unabhängig voneinander 0, 1, 2 oder 3,
das dadurch gekennzeichnet ist, dass es zusätzlich eine oder mehre Verbindungen der Formel S1, 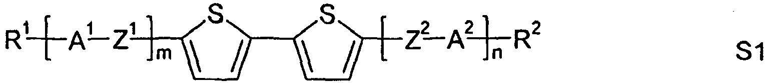 worin die einzelnen Reste folgende Bedeutung besitzen:
- R¹ und R²: H, F, Cl, Br, -CN, -SCN, -NCS, SF₅ oder geradkettiges oder verzweigtes Alkyl mit 1 bis 12 C-Atomen, worin auch eine oder mehrere nicht benachbarte CH₂-Gruppen jeweils unabhängig voneinander durch -CH=CH-, -C≡C-, -O-, -CO-, -CO-O-, -O-CO-, -O-CO-O- so ersetzt sein können, dass O-Atome nicht direkt miteinander verknüpft sind, und worin auch ein oder mehrere H-Atome durch F, Cl oder Br ersetzt sein können,
- A¹ und A²: jeweils unabhängig voneinander Phenylen-1,4-diyl, worin auch eine oder zwei CH-Gruppen durch N ersetzt sein können und ein oder mehrere H-Atome durch Halogen, CN, CH₃, CHF₂, CH₂F, OCH₃, OCHF₂ oder OCF₃ ersetzt sein können, Cyclohexan-1,4-diyl, worin auch eine oder zwei nicht benachbarte CH₂ -Gruppen unabhängig voneinander durch O und/oder S ersetzt sein können, und ein oder mehrere H-Atome durch F ersetzt sein können, Cyclohexen-1,4-diyl, Bicyclo[1.1.1]pentan-1,3-diyl, Bicyclo[2.2.2]octan-1,4-diyl, Spiro[3.3]heptan-2,6-diyl, Tetrahydropyran-2,5-diyl, oder 1,3-Dioxan-2,5-diyl,
- Z¹ und Z²: jeweils unabhängig voneinander -CF₂O-, -OCF₂-, -CH₂O-, -OCH₂-, -CO-O-, -O-CO-, -C₂H₄- -C₂F₄-, -CF₂CH₂-, - CH₂CF₂-, -CFHCFH-, -CFHCH₂-, -CH₂CFH-, -CF₂CFH-, - CFHCF₂-, -CH=CH-, -CF=CH-, -CH=CF-, -CF=CF-, -C≡C- oder eine Einfachbindung, und
- m und n: jeweils unabhängig voneinander 0, 1, 2 oder 3,
und/oder eine oder mehrere Verbindungen der Formel S2, 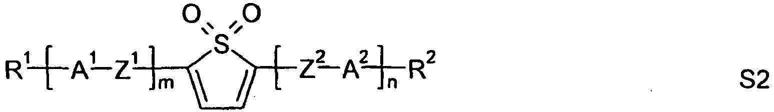 worin die einzelnen Reste folgende Bedeutung besitzen:
- R¹ und R²: H, F, Cl, Br, -CN, -SCN, -NCS, SF₅ oder geradkettiges oder verzweigtes Alkyl mit 1 bis 12 C-Atomen, worin auch eine oder mehrere nicht benachbarte CH₂-Gruppen jeweils unabhängig voneinander durch -CH=CH-, -C≡C-, -O-, -CO-, -CO-O-, -O-CO-, -O-CO-O- so ersetzt sein können, dass O-Atome nicht direkt miteinander verknüpft sind, und worin auch ein oder mehrere H-Atome durch F, Cl oder Br ersetzt sein können,
- A¹ und A²: jeweils unabhängig voneinander Phenylen-1,4-diyl, worin auch eine oder zwei CH-Gruppen durch N ersetzt sein können und ein oder mehrere H-Atome durch Halogen, CN, CH₃, CHF₂, CH₂F, OCH₃, OCHF₂ oder OCF₃ ersetzt sein können, Cyclohexan-1,4-diyl, worin auch eine oder zwei nicht benachbarte CH₂ -Gruppen unabhängig voneinander durch O und/oder S ersetzt sein können, und ein oder mehrere H-Atome durch F ersetzt sein können, Cyclohexen-1,4-diyl, Bicyclo[1.1.1]pentan-1,3-diyl, Bicyclo[2.2.2]octan-1,4-diyl, Spiro[3.3]heptan-2,6-diyl, Tetrahydropyran-2,5-diyl, oder 1,3-Dioxan-2,5-diyl,
- Z¹ und Z²: jeweils unabhängig voneinander -CF₂O-, -OCF₂-, -CH₂O-, -OCH₂-, -CO-O-, -O-CO-, -C₂H₄- -C₂F₄-, -CF₂CH₂-, - CH₂CF₂-, -CFHCFH-, -CFHCH₂-, -CH₂CFH-, -CF₂CFH-, - CFHCF₂-, -CH=CH-, -CF=CH-, -CH=CF-, -CF=CF-, -C≡C- oder eine Einfachbindung,
- m und n: jeweils unabhängig voneinander 0, 1, 2 oder 3,
enthält.

Ein weiterer Gegenstand der Erfindung sind die Verbindungen der Formel S2. Bevorzugt sind solche Verbindungen der Formel S2, die auch für die übrigen Ausführungsformen der Erfindung als bevorzugt angegeben sind.

Ein weiterer Gegenstand der Erfindung ist die Verwendung von Verbindungen der Formel S1 und/oder der Formel S2, bevorzugt beide, in FK-Medien oder elektrooptischen Vorrichtungen mit Thiophenverbindungen der Formel I, insbesondere in FK-Anzeigen, sowie die Verwendung von erfindungsgemäßen FK-Medien in solchen Anzeigen.

Ein weiterer Gegenstand der Erfindung ist eine FK-Anzeige enthaltend eine oder mehrere Verbindungen der Formel I, eine oder mehrere Verbindungen der Formel S1, und optional eine oder mehrere Verbindungen der Formel S2 oder eine Anzeige enthaltend ein erfindungsgemäßes FK-Medium, insbesondere eine TN-, VA- oder IPS-Anzeige.

FK-Medien, die ohne Gegenwart von chiralen Dotierstoffen eine achirale FK-Phase aufweisen, sowie Verbindungen der Formel I, worin die Reste Z^{1,2}, A^{1,2}, R^{1,2} kein Chiralitätszentrum aufweisen, sind generell bevorzugt.

Bevorzugt sind Medien enthaltend die Verbindungen der Formel I, worin A⁰ ein Phenylen-1,4-diyl, worin auch eine oder zwei CH-Gruppen durch N ersetzt sein können und ein oder mehrere H-Atome durch Halogen, CN, CH₃, CHF₂, CH₂F, OCH₃, OCHF₂ oder OCF₃ ersetzt sein können, bedeutet,
besonders bevorzugt, worin
A⁰ 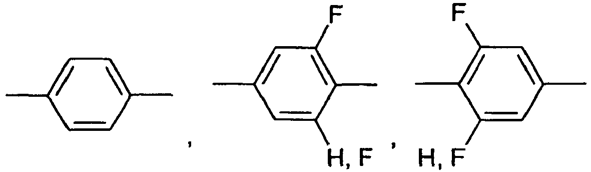 oder 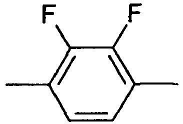 und ganz besonders bevorzugt, worin
A⁰ 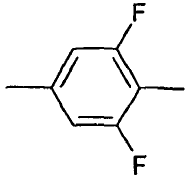 bedeutet.

Die bevorzugten Verbindungen der Formel I führen zu Medien mit besonders hohem Klärpunkt, geringer Rotationsviskosität, breiter nematischer Phase, hoher Doppelbrechung und guter Stabilität.

Ferner bevorzugt sind Verbindungen der Formel I, worin m und n 0, 1 oder 2, besonders bevorzugt 0 oder 1 bedeuten. Besonders bevorzugt sind Verbindungen der Formel I, worin n 0 bedeutet, also der Thiophenring ein endständiger Ring ist, und m 1, 2 oder mehr bedeutet. Weiterhin bevorzugt sind Verbindungen der Formel I, worin m 0, 1 oder 2, vorzugsweise 1 oder 2, und ganz besonders bevorzugt 2 bedeutet.

A¹ und A² in Formel I, S1 und S2 bedeuten besonders bevorzugt Phenylen-1,4-diyl, welches auch ein oder mehrfach durch F substituiert sein kann, ferner Cyclohexan-1,4-diyl, Tetrahydropyran-2,5-diyl, oder 1,3-Dioxan-2,5-diyl.

Z¹ und Z² in Formel I, S1 und S2 bedeuten besonders bevorzugt -CF₂O-, -OCF₂- oder eine Einfachbindung, insbesondere eine Einfachbindung.

Besonders bevorzugt bedeuten A¹ und A² in Formel I, S1 und S2 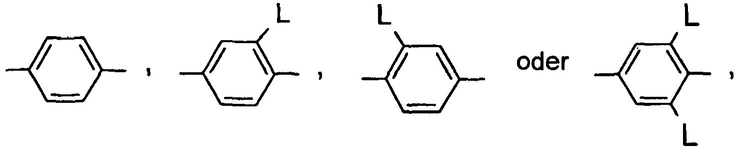 bevorzugt unsubstituiertes 1,4-Phenylen,
worin L Halogen, CF₃ oder CN, vorzugsweise F bedeutet.

Ferner bevorzugt sind Verbindungen der Formel I, worin
R¹ und R² jeweils unabhängig voneinander H, F, Cl, Br, -CN, -SCN, -NCS, SF₅, Halogen oder optional durch Halogen, insbesondere durch F, substituiertes Alkyl, Alkenyl oder Alkinyl mit 1 bis 8, vorzugsweise 1 bis 5 C-Atomen bedeuten.

Besonders bevorzugte Reste R¹ und R² in Formel I, S1 und S2 bedeuten H, Halogen, oder optional durch Halogen, insbesondere durch F, substituiertes Alkyl, Alkenyl, Alkinyl oder Alkoxy mit 1 bis 12, vorzugsweise 1 bis 8 C-Atomen, besonders bevorzugt H, F, Alkyl, Alkenyl oder Alkinyl mit 1 bis 8 C-Atomen. Bevorzugt bedeutet wenigstens ein Rest kein H, besonders bevorzugt sind beide Reste R¹ und R² nicht H. Ganz besonders bevorzugt ist R¹ gleich Alkyl. Ferner bevorzugt ist R² H, Alkyl oder Fluor. Ganz besonders bevorzugt ist R¹ Alkyl und R² H oder Alkyl. R¹, R² bedeuten jeweils unabhängig voneinander ganz besonders bevorzugt unverzweigtes Alkyl mit 1-5 C-Atomen. Falls R¹ und R² substituiertes Alkyl, Alkoxy, Alkenyl oder Alkinyl bedeuten, beträgt die Gesamtzahl der C-Atome in beiden Gruppen R¹ und R² vorzugsweise weniger als 10.

Bevorzugte Alkylgruppen sind beispielsweise Methyl, Ethyl, n-Propyl, n-Butyl, n-Pentyl, n-Hexyl, n-Heptyl und n-Octyl.

Bevorzugte Alkenylgruppen sind beispielsweise Ethenyl, Propenyl, Butenyl und Pentenyl.

Bevorzugte Alkinylgruppen sind beispielsweise Ethinyl, Propinyl, Butinyl, Pentinyl, Hexinyl, Heptinyl und Octinyl.

Bevorzugte Alkoxygruppen sind beispielsweise Methoxy, Ethoxy, n-Propoxy, n-Butoxy, n-Pentoxy, n-Hexoxy, n-Heptoxy, n-Octoxy.

Halogen bedeutet vorzugsweise F oder Cl.

Besonders bevorzugte Verbindungen der Formel I sind solche ausgewählt aus folgenden Unterformeln 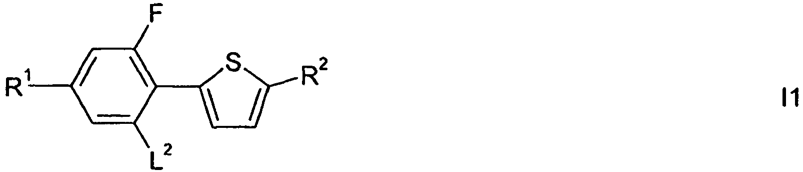 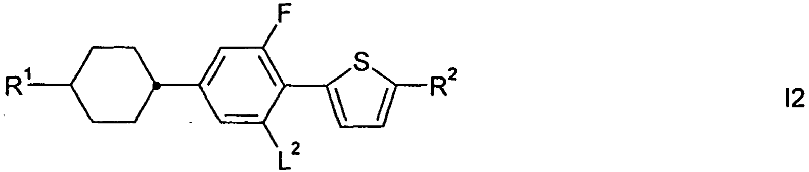 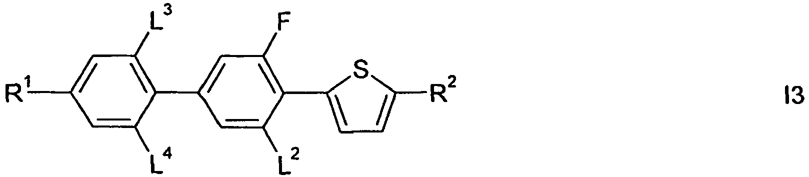 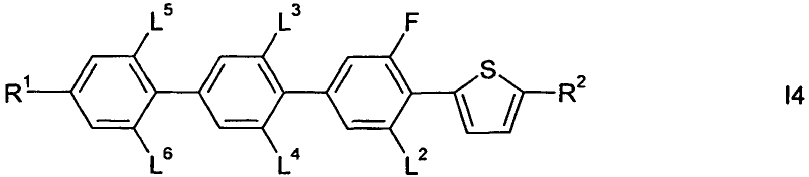 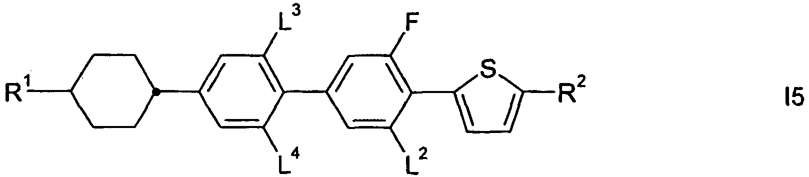 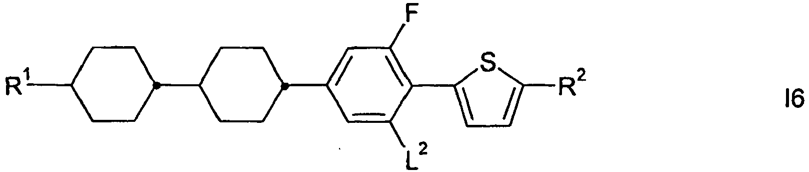 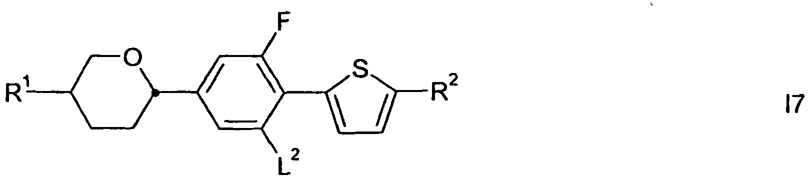 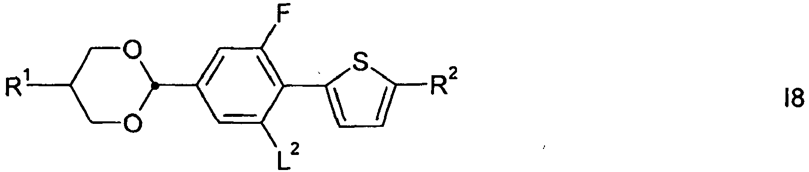 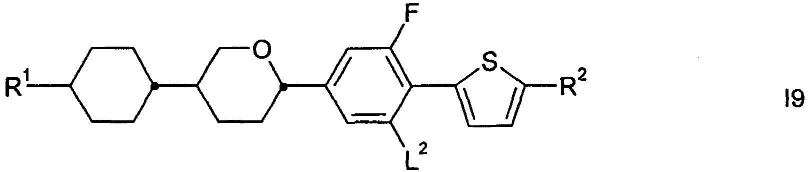 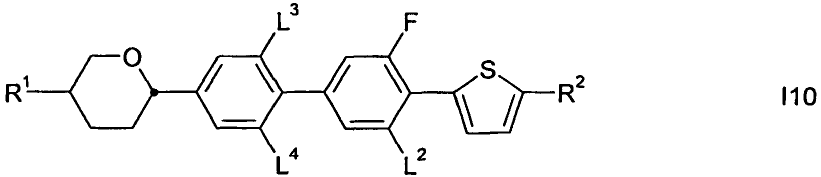 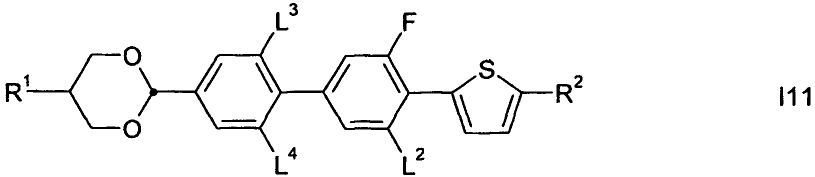 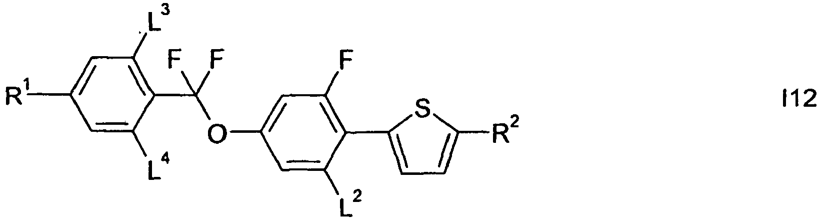 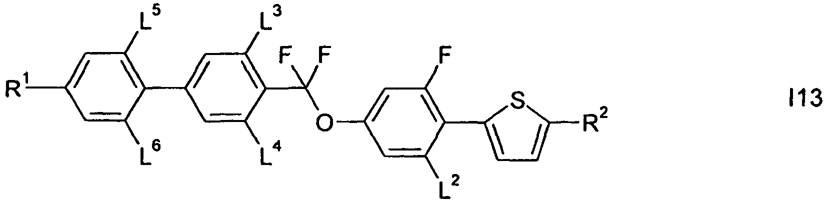 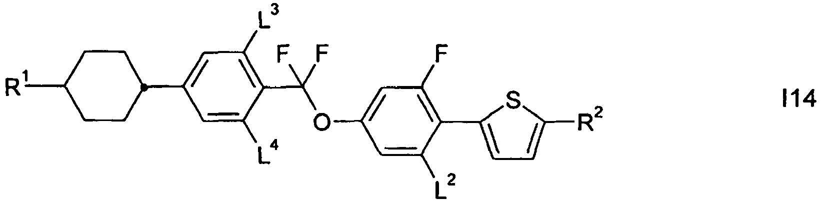 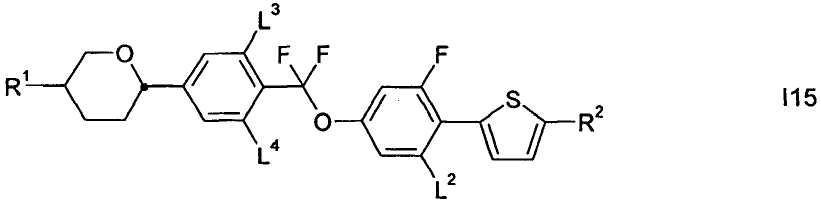 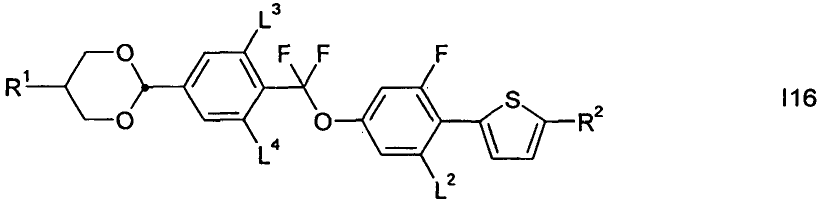 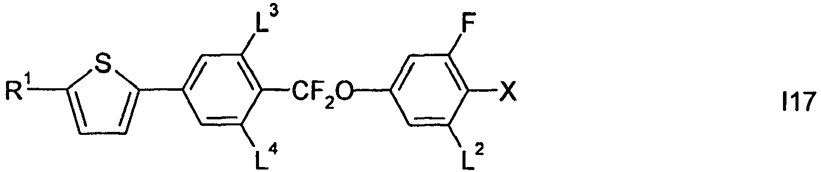 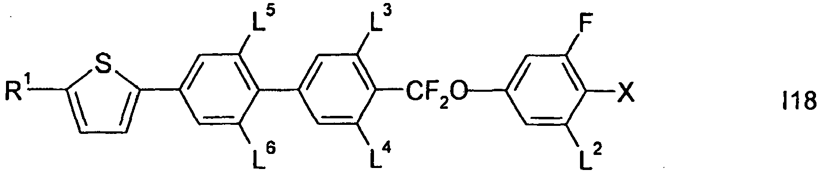 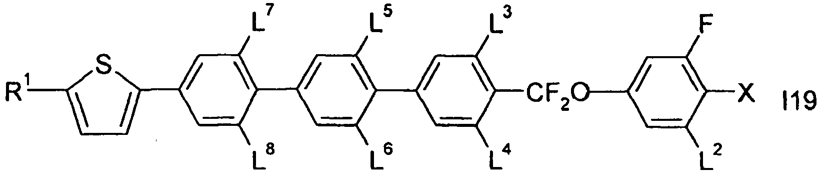 worin R¹ und R² die vor- und nachstehend angegebenen Bedeutungen besitzen, und L¹ bis L⁸ unabhängig H oder F bedeuten. R¹ und R² bedeuten darin vorzugsweise optional fluoriertes Alkyl, Alkenyl, Alkinyl oder Alkoxy mit 1 bis 12 C-Atomen, besonders bevorzugt optional fluoriertes Alkyl, Alkenyl oder Alkinyl mit 1 bis 5 C-Atomen. L² bedeutet in den Formeln bevorzugt F. In den Formeln 11 bis I11 bedeuten L³ und L⁴ bevorzugt H. In den Formeln I12 bis I19 bedeuten L³ und L⁴ bevorzugt F. Bevorzugte erfindungsgemäße Medien enthalten Verbindungen ausgewählt aus den Formel 13 und I17.

Die Verbindungen der Formel I können in Analogie zu dem Fachmann bekannten und in Standardwerken der organischen Chemie beschriebenen Verfahren, wie beispielsweise in Houben-Weyl, Methoden der organischen Chemie, Thieme-Verlag, Stuttgart, hergestellt werden.

Die Synthese von geeigneten Thiophenen der Formel I ist bekannt, z.B. aus WO 2009/129915 A1, und den internationalen Anmeldungen PCT/EP2010/000636 und PCT/EP2010/000968.

Die Dithiophene S1 werden vorzugsweise durch Funktionalisierung von 2,2'-Bithiophen mittels Metallierung in 2-Position oder durch Suzuki-Kopplung zweier funktionalisierter Thiophenringe analog literaturbekannter Beispiele und Methoden dargestellt.

Die Synthese der Thiophenoxide S2 erfolgt vorzugsweise durch Oxidation entsprechender Thiophene, für die ausreichend Literaturbeispiele zur Herstellung existieren. Als Reagenz für diese Oxidation eignet sich besonders MCPBA (3-Chlorperoxybenzoesäure). Weitere Details können den Synthesebeispielen entnommen werden, die eine allgemein anwendbare Synthesemethode wiedergeben, die repräsentativ für alle Verbindungen der Formel S2 ist.

Bevorzugte Dithiophen-Verbindungen der Formel S1 sind dadurch gekennzeichnet, dass m und n 0, 1 oder 2, bevorzugt 0 oder 1, sind und m oder n den Wert 0 annehmen, besonders bevorzugt m und n = 0.

Besonders bevorzugte Verbindungen der Formel S2 sind solche ausgewählt aus folgenden Unterformeln S2-1 bis S2-2: 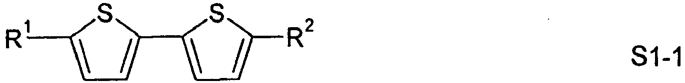 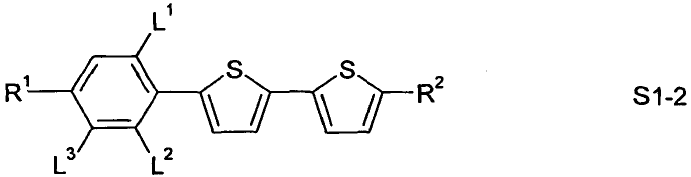 worin L¹⁻³ unabhängig voneinander H oder F bedeuten.

Bevorzugte Thienophenoxid-Verbindungen der Formel S2 sind dadurch gekennzeichnet, dass m und n 0, 1 oder 2 bedeuten. Besonders bevorzugt sind Verbindungen der Formel S2, worin n 0 bedeutet. Weiterhin bevorzugt sind Verbindungen der Formel S2, worin m 1 oder 2, besonders bevorzugt 2 bedeutet.

Bevorzugte Verbindungen der Formel S2 sind dadurch gekennzeichnet, dass Z¹ und Z² Einfachbindungen sind, oder dass eine Gruppe aus Z^{1/2} eine Einfachbindung und die andere eine Gruppe -CF₂O- oder -OCF₂-bedeutet.

Besonders bevorzugte Verbindungen der Formel S2 sind solche ausgewählt aus folgenden Unterformeln S2-1 bis S2-7: 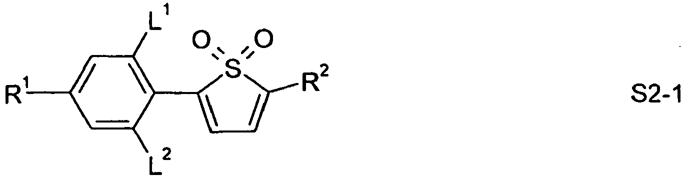 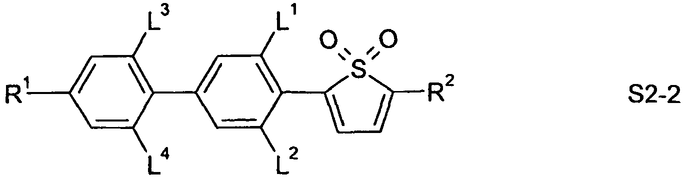 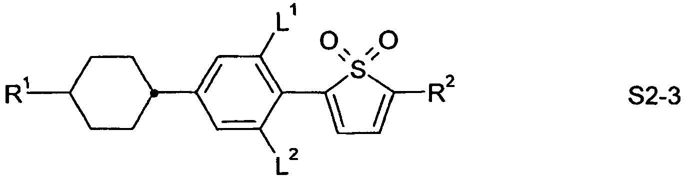 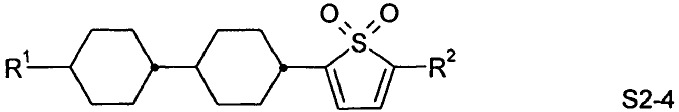 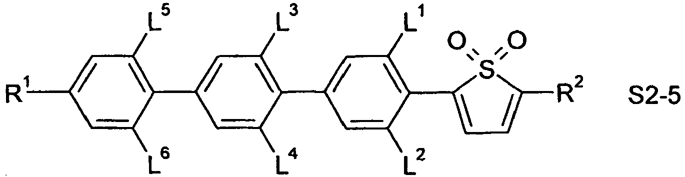 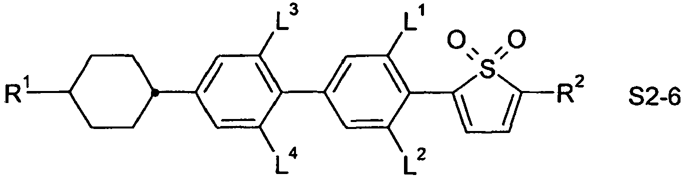 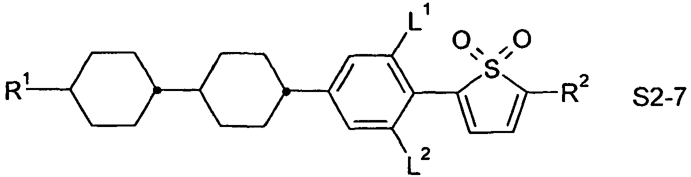 worin L¹⁻⁶ unabhängig voneinander H oder F bedeuten.

Besonders bevorzugte erfindungsgemäße FK-Medien werden im Folgenden genannt:
- FK-Medium, welches bevorzugte Verbindungen bezeichnete Verbindungen der Formel I, S1 und/oder S2 enthält.
- FK-Medium, welches zusätzlich eine oder mehrere Verbindungen der Formel II und/oder III enthält: 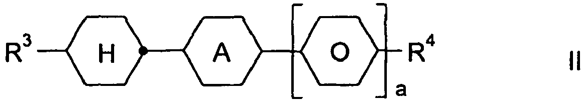 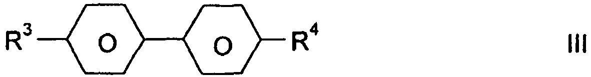 worin
   - A: 1,4-Phenylen oder trans-1,4-Cyclohexylen bedeutet,
   - a: 0 oder 1 ist,
   - R³: Alkyl oder Alkenyl mit bis zu 9 C-Atomen, vorzugsweise Alkenyl mit 2 bis 9 C-Atomen bedeutet, und
   - R⁴: Alkyl mit 1 bis 12 C-Atomen, wobei auch eine oder zwei nicht benachbarte CH₂-Gruppen durch -O-, -CH=CH-, -CO-, -OCO- oder -COO- so ersetzt sein können, dass O-Atome nicht direkt miteinander verknüpft sind, und vorzugsweise Alkyl mit 1 bis 12 C-Atomen oder Alkenyl mit 2 bis 9 C-Atomen bedeutet.
   Die Verbindungen der Formel II sind vorzugsweise ausgewählt aus der Gruppe bestehend aus folgenden Formeln: 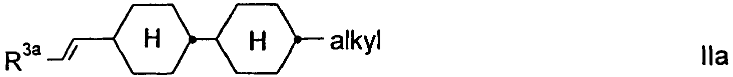 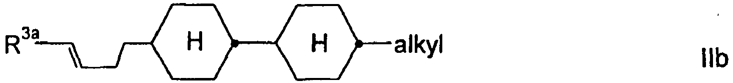 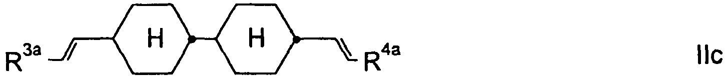 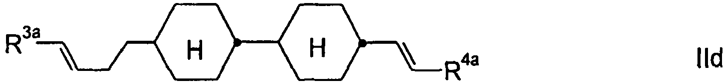 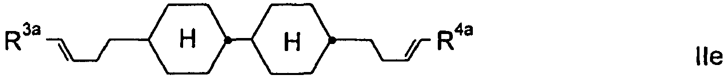 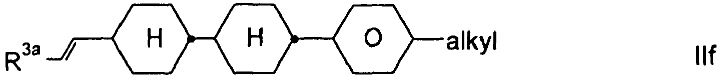 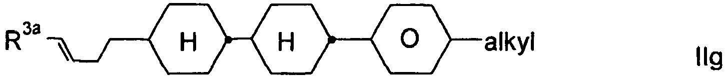 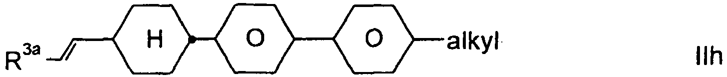 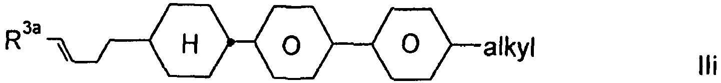 worin R^{3a} und R^{4a} jeweils unabhängig voneinander H, CH₃, C₂H₅ oder C₃H₇ bedeuten, und "alkyl" eine geradkettige Alkylgruppe mit 1 bis 8, vorzugsweise 1, 2, 3, 4 oder 5 C-Atomen bedeutet. Besonders bevorzugt sind Verbindungen der Formel IIa und IIf, insbesondere worin R^{3a} H oder CH₃ , vorzugsweise H, bedeutet, und Verbindungen der Formel IIc, insbesondere worin R³¹ und R^{4a} H, CH₃ oder C₂H₅ bedeuten.
   Die Verbindungen der Formel III sind vorzugsweise ausgewählt aus der Gruppe bestehend aus den folgenden Formeln: 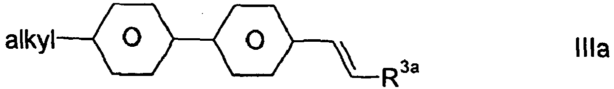 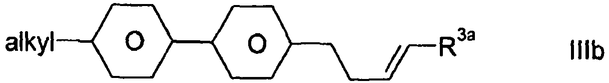 worin "alkyl" und R^{3a} die oben angegebenen Bedeutungen haben und R^{3a} vorzugsweise H oder CH₃ bedeutet. Besonders bevorzugt sind Verbindungen der Formel IIIb;
- FK-Medium, welches zusätzlich eine oder mehrere Verbindungen ausgewählt aus der Gruppe bestehend aus den folgenden Formeln enthält: 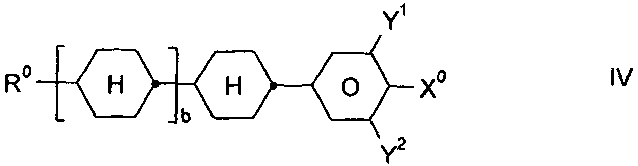 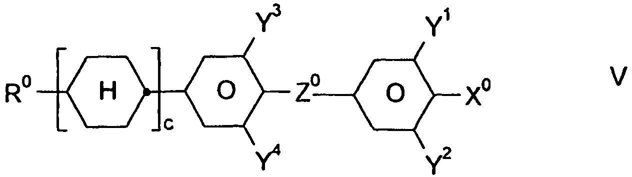 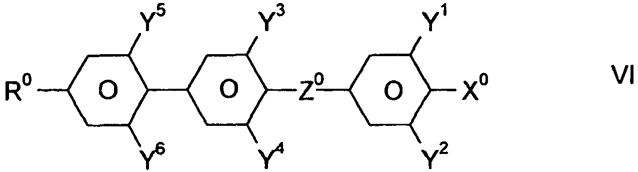 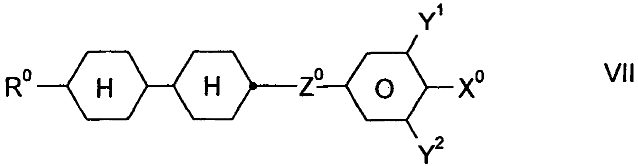 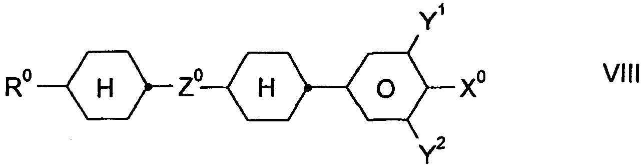 worin
   - R⁰: einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O- -CH=CH-, 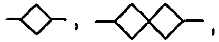 -O-, -CO-O- oder -O-CO- so ersetzt sein können, dass O-Atome nicht direkt miteinander verknüpft sind, und worin auch ein oder mehrere H-Atome durch Halogen ersetzt sein können,
   - X⁰: F, Cl, CN, SF₅, SCN, NCS, einen halogenierten Alkylrest, halogenierten Alkenylrest, halogenierten Alkoxyrest oder halogenierten Alkenyloxyrest mit jeweils bis zu 6 C-Atomen,
   - Y¹⁻⁶: jeweils unabhängig voneinander H oder F,
   - Z⁰: -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- oder -OCF₂-, in den Formeln V und VI auch eine Einfachbindung, und
   - b und c: jeweils unabhängig voneinander 0 oder 1
   bedeuten.
   In den Verbindungen der Formel IV bis VIII bedeutet X⁰ vorzugsweise F oder OCF₃, ferner OCHF₂, CF₃, CF₂H, Cl, OCH=CF₂. R⁰ ist vorzugsweise geradkettiges Alkyl oder Alkenyl mit jeweils bis zu 6 C-Atomen.
   Die Verbindungen der Formel IV sind vorzugsweise ausgewählt aus der Gruppe bestehend aus den folgenden Formeln: 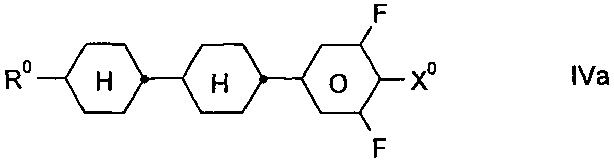 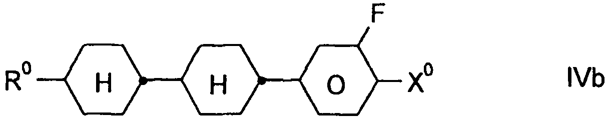 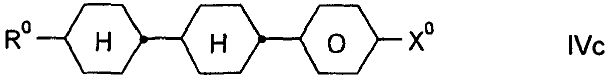 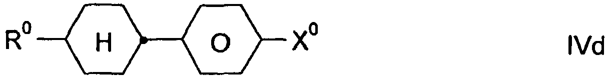 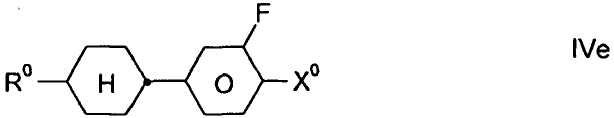 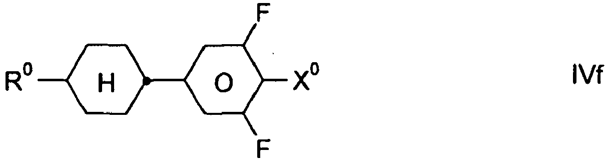 worin R⁰ und X⁰ die oben angegebenen Bedeutungen haben.
   Vorzugsweise bedeutet in Formel IV R⁰ Alkyl mit 1 bis 8 C-Atomen und X⁰ F, Cl, OCHF₂ oder OCF₃, ferner OCH=CF₂. In der Verbindung der Formel IVb bedeutet R⁰ vorzugsweise Alkyl oder Alkenyl. In der Verbindung der Formel IVd bedeutet X⁰ vorzugsweise Cl, ferner F.
   Die Verbindungen der Formel V sind vorzugsweise ausgewählt aus der Gruppe bestehend aus den folgenden Formeln: 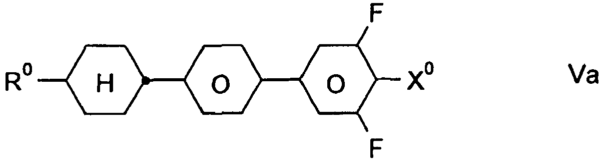 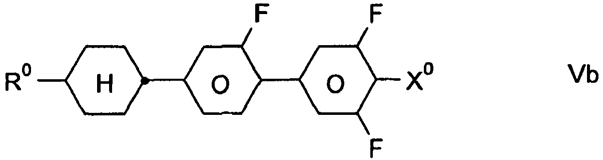 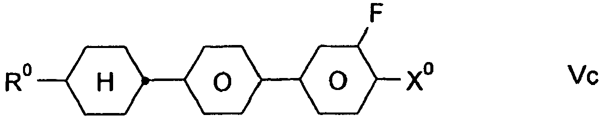 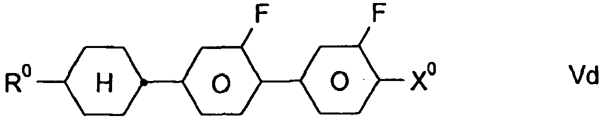 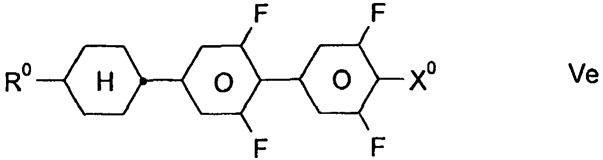 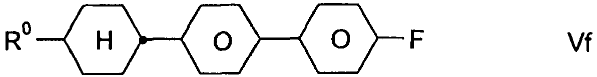 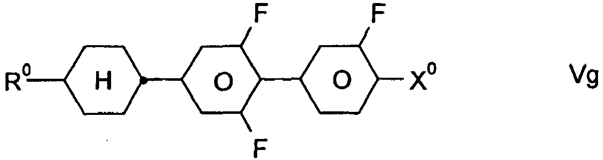 worin R⁰ und X⁰ die oben angegebenen Bedeutungen haben. Vorzugsweise bedeutet R⁰ in Formel V Alkyl mit 1 bis 8 C-Atomen und X⁰ F;
- FK-Medium, welches eine oder mehrere Verbindungen der Formel VI-1 enthält: 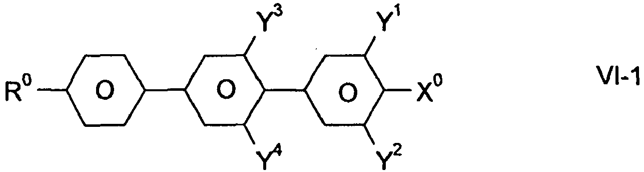 worin Y¹ bevorzugt F bedeutet,
   besonders bevorzugt solche ausgewählt aus der Gruppe bestehend aus den folgenden Formeln: 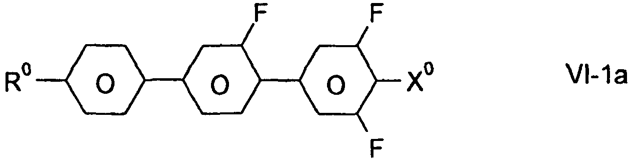 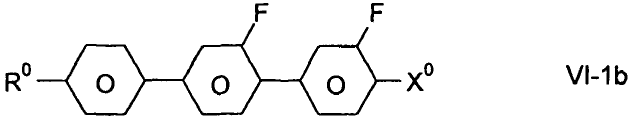 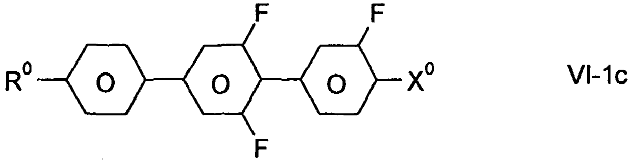 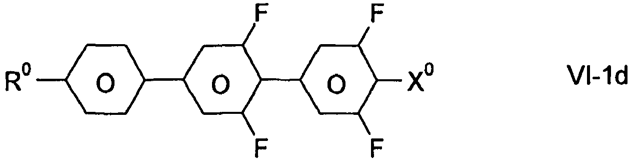 worin R⁰ und X⁰ die oben angegebenen Bedeutungen haben. Vorzugsweise bedeutet R⁰ in Formel VI Alkyl mit 1 bis 8 C-Atomen und X⁰ F, ferner OCF₃.
- FK-Medium, welches eine oder mehrere Verbindungen der Formel VI-2 enthält: 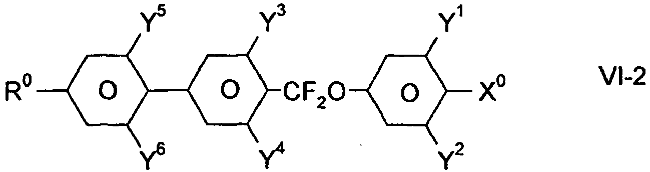 besonders bevorzugt solche ausgewählt aus der Gruppe bestehend aus den folgenden Formeln: 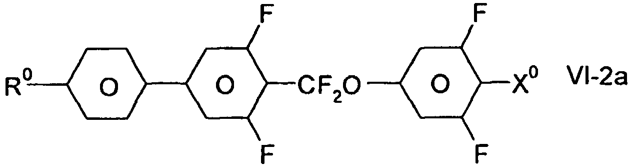 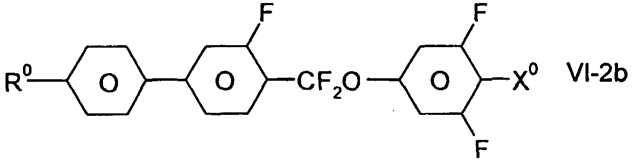 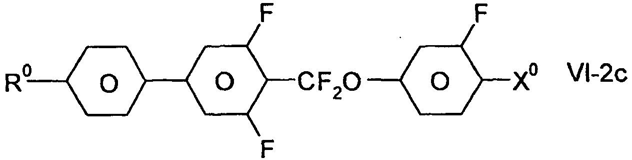 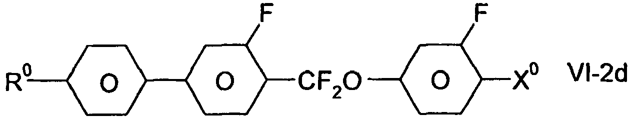 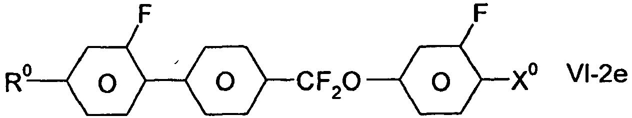 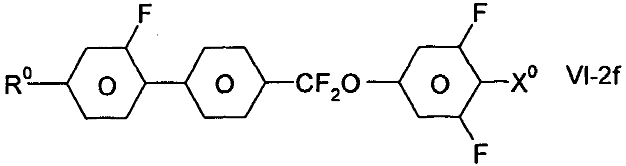 worin R⁰ und X⁰ die oben angegebenen Bedeutungen haben.
   Vorzugsweise bedeutet R⁰ in Formel VI Alkyl mit 1 bis 8 C-Atomen und X⁰F;
- FK-Medium, welches vorzugsweise eine oder mehrere Verbindungen der Formel VII, worin Z⁰ -CF₂O- -CH₂CH₂ oder -COO-, bedeutet, enthält, besonders bevorzugt solche ausgewählt aus der Gruppe bestehend aus den folgenden Formeln: 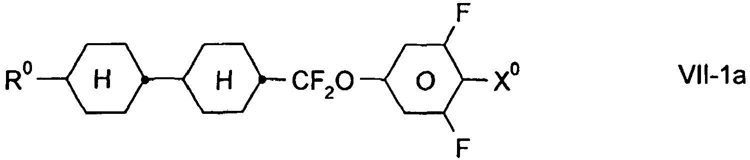 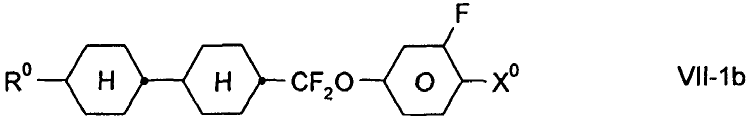 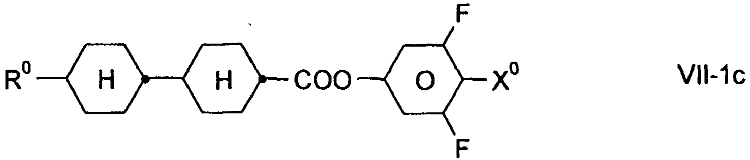 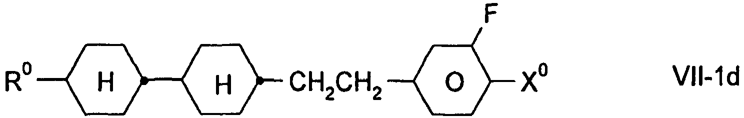 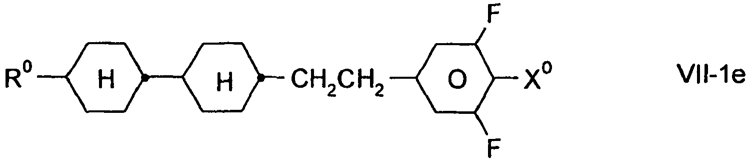 worin R⁰ und X⁰ die oben angegebenen Bedeutungen haben. Vorzugsweise bedeutet R⁰ in Formel VII Alkyl mit 1 bis 8 C-Atomen und X⁰ F, ferner OCF₃.
   Die Verbindungen der Formel VIII sind vorzugsweise ausgewählt aus der Gruppe bestehend aus den folgenden Formeln: 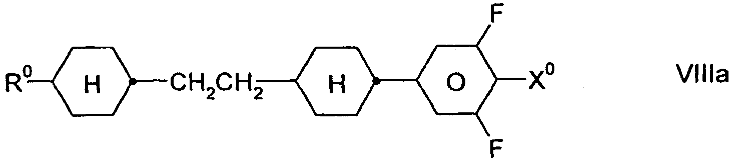 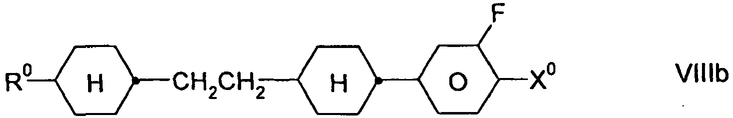 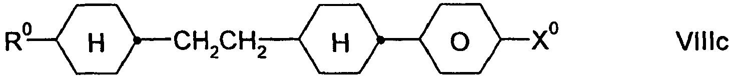 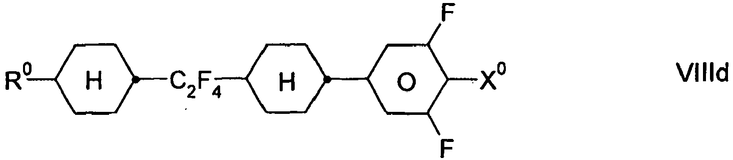 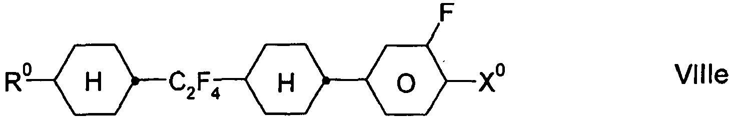 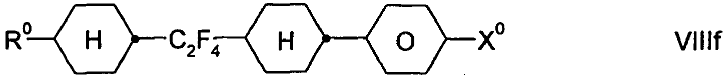 worin R⁰ und X⁰ die oben angegebenen Bedeutungen haben. Vorzugsweise bedeutet R⁰ einen geradkettigen Alkylrest mit 1 bis 8 C-Atomen. X⁰ bedeutet vorzugsweise F.
- FK-Medium, welches zusätzlich eine oder mehrere Verbindungen der folgenden Formel enthält: 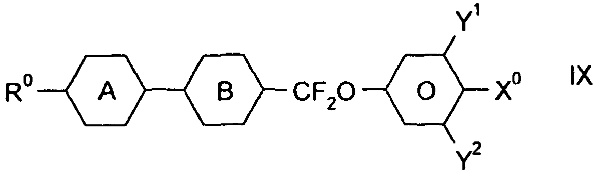 worin R⁰, X⁰, Y¹ und Y² die oben angegebene Bedeutung besitzen, und 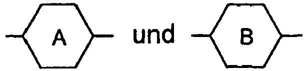 jeweils unabhängig voneinander 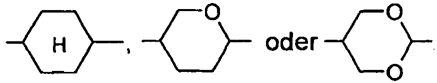 bedeuten, wobei die Ringe A und B nicht beide gleichzeitig Cyclohexylen bedeuten;
   Die Verbindungen der Formel IX sind vorzugsweise ausgewählt aus der Gruppe bestehend aus den folgenden Formeln: 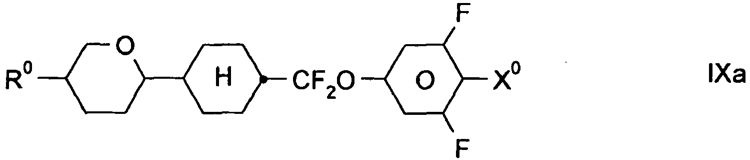 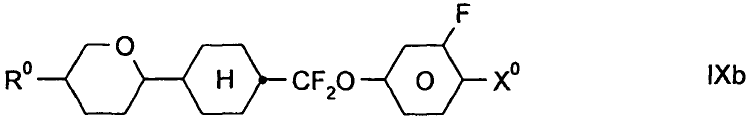 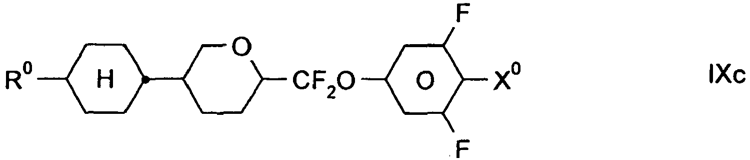 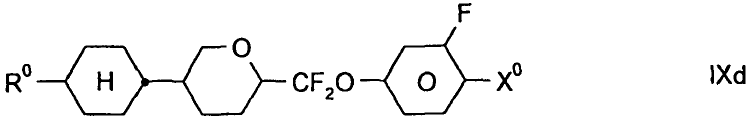 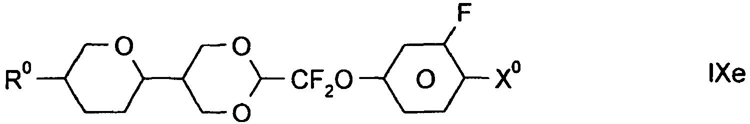 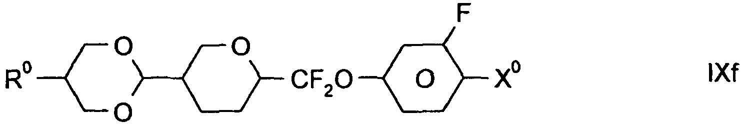 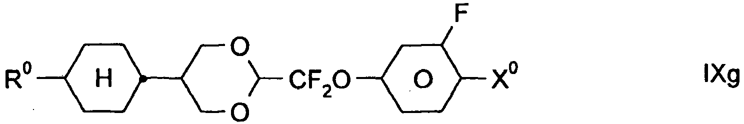 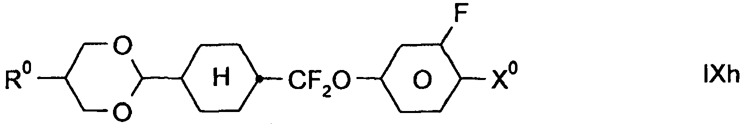 worin R⁰ und X⁰ die oben angegebenen Bedeutungen haben. Vorzugsweise bedeutet R⁰ Alkyl mit 1 bis 8 C-Atomen und X⁰ F. Besonders bevorzugt sind Verbindungen der Formel IXa;
- FK-Medium, welches zusätzlich eine oder mehrere Verbindungen ausgewählt aus der Gruppe bestehend aus den folgenden Formeln enthält: 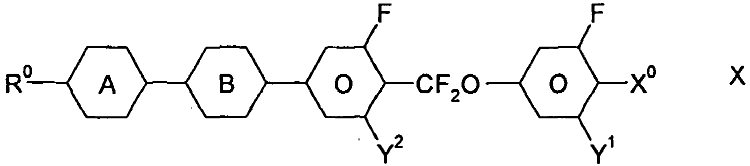 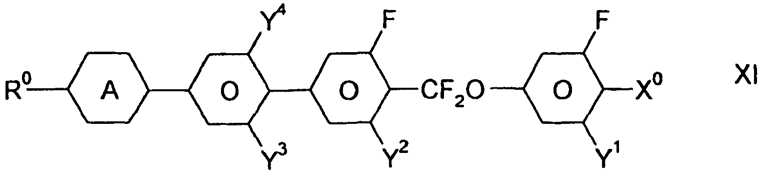 worin R⁰, X⁰ und Y¹⁻⁴ die oben angegebenen Bedeutungen besitzen, und 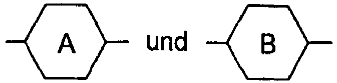 jeweils unabhängig voneinander 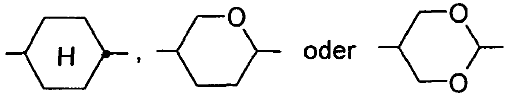 bedeuten;
   Die Verbindungen der Formeln X und XI sind vorzugsweise ausgewählt aus der Gruppe bestehend aus den folgenden Formeln: 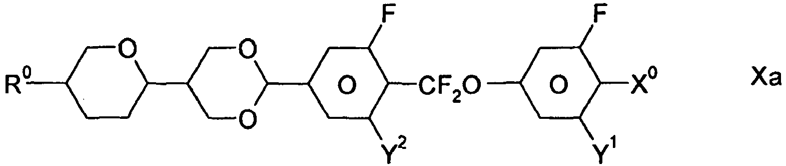 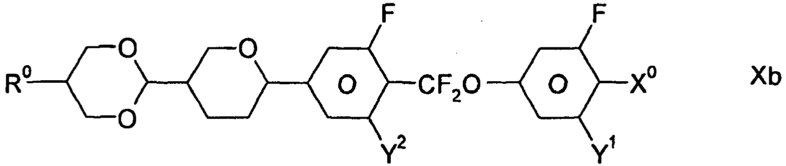 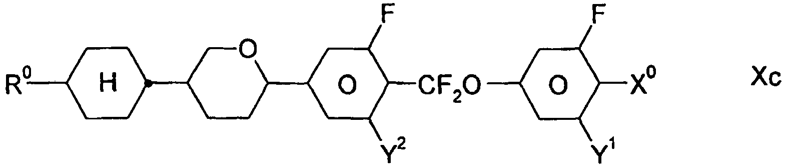 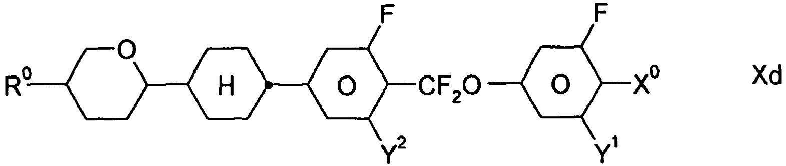 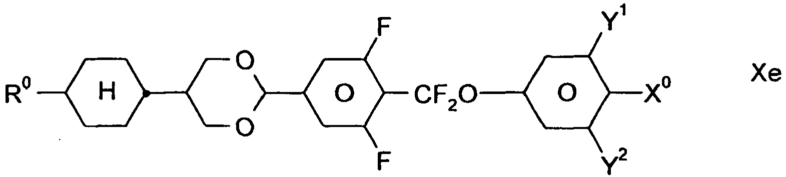 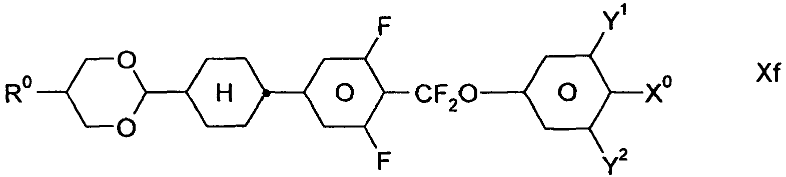 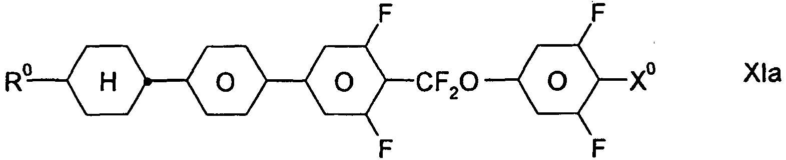 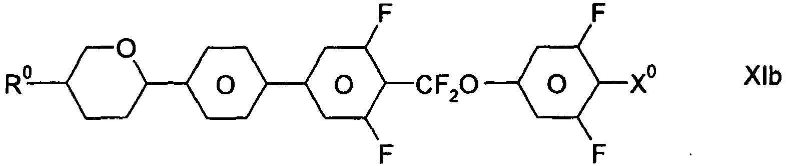 worin R⁰ und X⁰ die oben angegebenen Bedeutungen haben. Vorzugsweise bedeutet R⁰ Alkyl mit 1 bis 8 C-Atomen und X⁰ F. Besonders bevorzugte Verbindungen sind solche, worin Y¹ F und Y² H oder F, vorzugsweise F, bedeuten;
- FK-Medium, welches zusätzlich eine oder mehrere Verbindungen der folgenden Formel enthält: 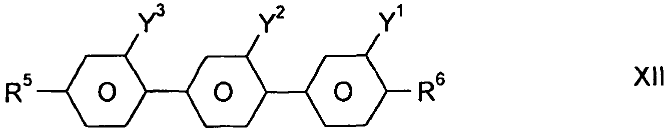 worin R⁵ und R⁶ jeweils unabhängig voneinander n-Alkyl, Alkoxy, Oxaalkyl, Fluoralkyl oder Alkenyl mit jeweils bis zu 9 C-Atomen bedeuten, und vorzugsweise jeweils unabhängig voneinander Alkyl mit 1 bis 8 C-Atomen bedeuten. Y¹⁻³ bedeutet unabhängig voneinander H oder F. Vorzugsweise bedeuten ein oder zwei der Gruppen aus Y¹⁻³ F.
   Bevorzugte Verbindungen der Formel XII sind solche ausgewählt aus der Gruppe bestehend aus den folgenden Formeln: 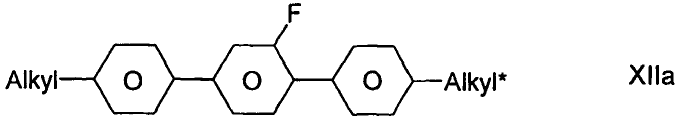 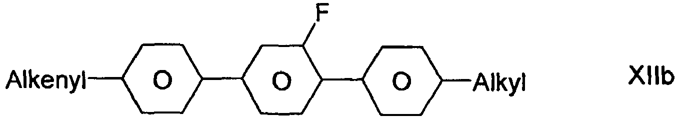 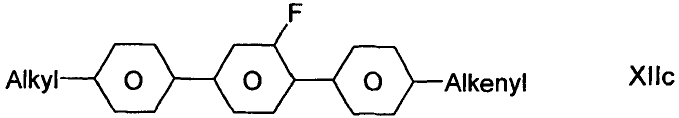 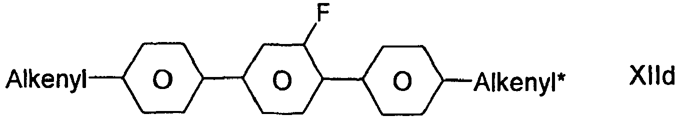 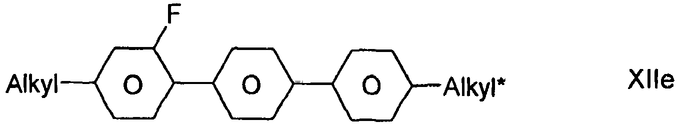 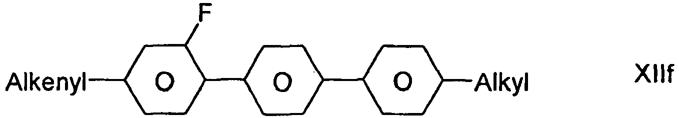 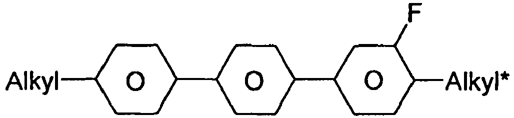 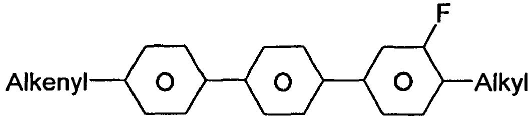 worin
   - Alkyl und Alkyl*: jeweils unabhängig voneinander einen geradkettigen Alkylrest mit 1 bis 6 C-Atomen, und
   - Alkenyl und Alkenyl*: jeweils unabhängig voneinander einen geradkettigen Alkenylrest mit 2 bis 6 C-Atomen
   bedeuten.
   Ganz besonders bevorzugt sind Verbindungen der folgenden Formel 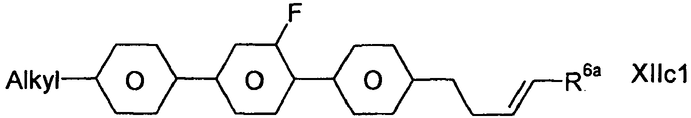 worin Alkyl die oben angegebene Bedeutung hat und R^{6a} H oder CH₃ bedeutet.
- FK-Medium, welches zusätzlich eine oder mehrere Verbindungen ausgewählt aus der Gruppe bestehend aus den folgenden Formeln enthält: 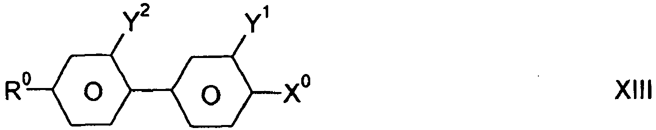 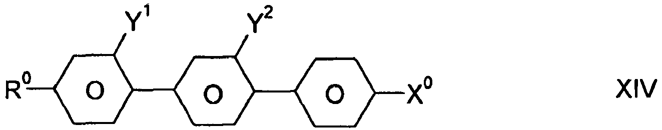 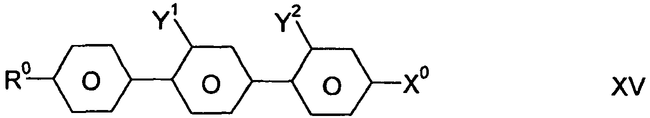 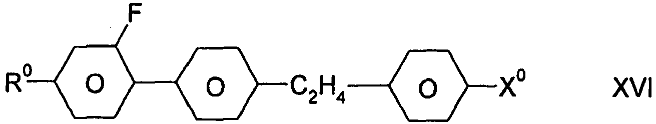 worin R⁰, X⁰, Y¹ und Y² die oben angegebenen Bedeutungen haben. Vorzugsweise bedeutet R⁰ Alkyl mit 1 bis 8 C-Atomen und X⁰ F oder Cl;
   Die Verbindungen der Formeln XIII und XIV sind vorzugsweise ausgewählt aus der Gruppe bestehend aus den folgenden Formeln: 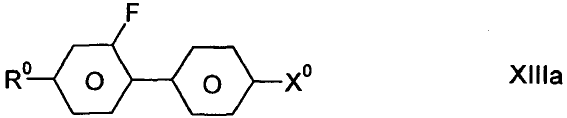 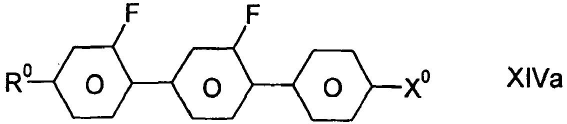 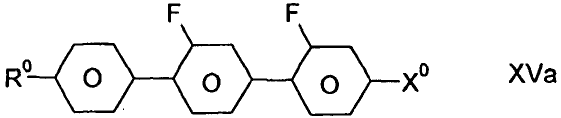 worin R⁰ und X⁰ die oben angegebenen Bedeutungen haben. Vorzugsweise bedeutet R⁰ Alkyl mit 1 bis 8 C-Atomen. In den Verbindungen der Formel XIII bedeutet X⁰ vorzugsweise F oder Cl.
- FK-Medium, welches zusätzlich eine oder mehrere Verbindungen der Formel D1 und/oder D2 enthält: 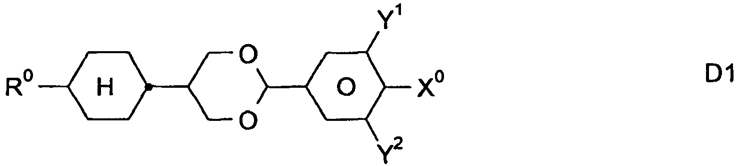 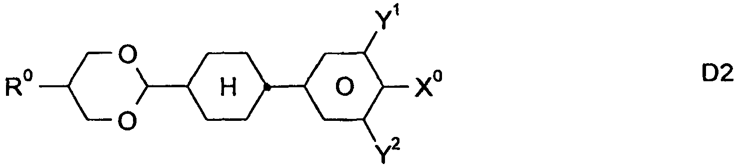 worin Y¹, Y², R⁰ und X⁰ die oben angegebene Bedeutung besitzen. Vorzugsweise bedeutet R⁰ Alkyl mit 1 bis 8 C-Atomen und X⁰ F. Besonders bevorzugt sind Verbindungen der folgenden Formeln: 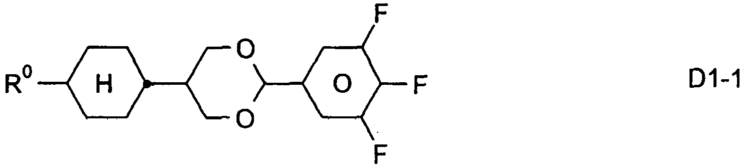 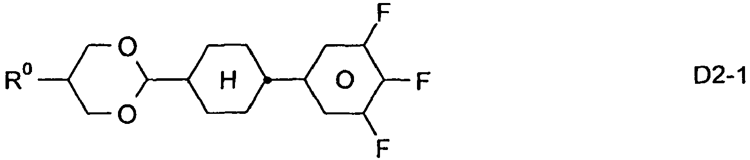 worin R⁰ die oben angegebenen Bedeutungen hat und vorzugsweise geradkettiges Alkyl mit 1 bis 6 C-Atomen, insbesondere C₂H₅, n-C₃H₇ oder n-C₅H₁₁bedeutet.
- FK-Medium, welches zusätzlich eine oder mehrere Verbindungen der folgenden Formel enthält: 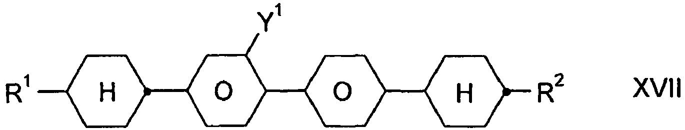 worin Y¹, R¹ und R² die oben angegebene Bedeutung besitzen. R¹ und R² bedeuten vorzugsweise jeweils unabhängig voneinander Alkyl mit 1 bis 8 C-Atomen;
- FK-Medium, welches zusätzlich eine oder mehrere Verbindungen der folgenden Formel enthält: 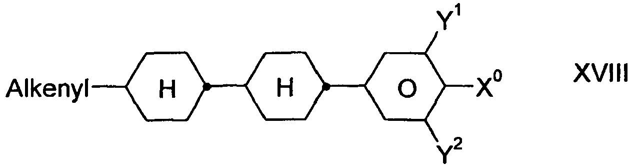 worin X⁰, Y¹ und Y² die oben angegebenen Bedeutungen besitzen und "Alkenyl" C₂₋₇-Alkenyl bedeutet. Besonders bevorzugt sind Verbindungen der folgenden Formel, 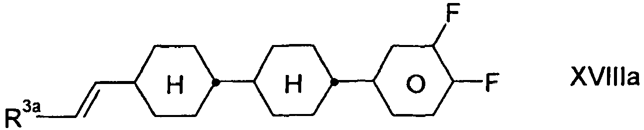 worin R^{3a} die oben angegebene Bedeutung hat und vorzugsweise H bedeutet;
- FK-Medium, welches zusätzlich eine oder mehrere Vierkern-Verbindungen ausgewählt aus der Gruppe bestehend aus den Formeln XIX bis XXV enthält: 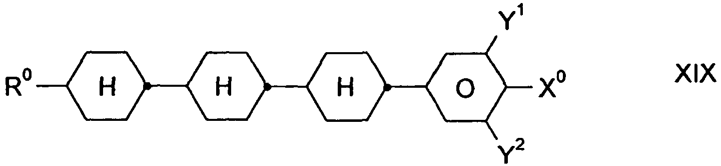 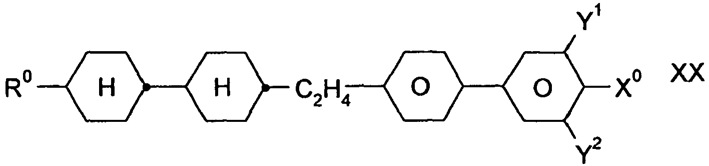 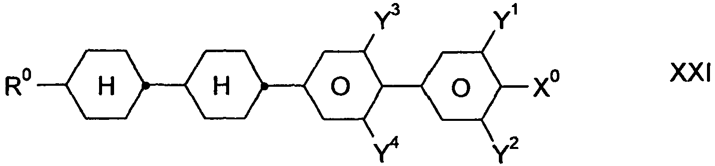 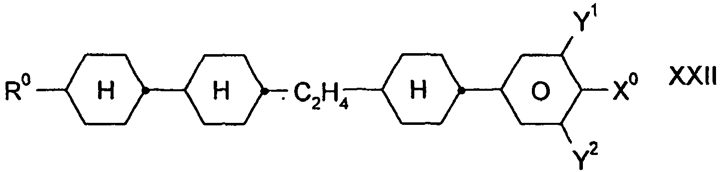 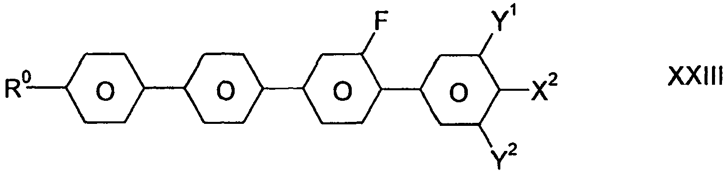 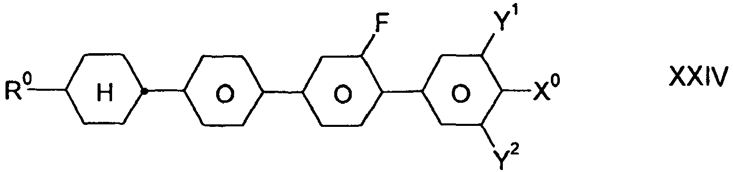 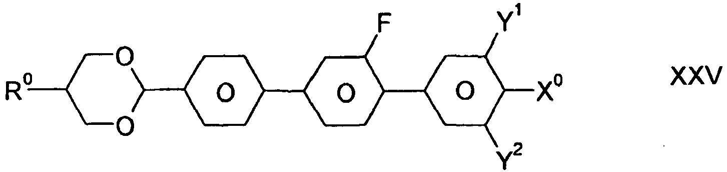 worin Y¹⁻⁴, R⁰ und X⁰ jeweils unabhängig voneinander eine der oben angegebenen Bedeutungen haben. X⁰ ist vorzugsweise F, Cl, CF₃, OCF₃ oder OCHF₂. R⁰ bedeutet vorzugsweise Alkyl, Alkoxy, Oxaalkyl, Fluoralkyl oder Alkenyl mit jeweils bis zu 8 C-Atomen.
- FK-Medium, welches zusätzlich eine oder mehrere Verbindungen der folgenden Formel enthält: 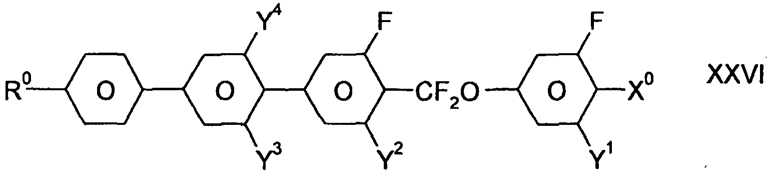 worin R⁰, X⁰ und Y¹⁻⁴ die oben angegebenen Bedeutungen besitzen. Besonders bevorzugt sind Verbindungen der folgenden Formel: 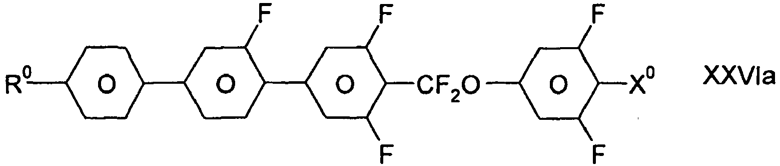
- FK-Medium, welches zusätzlich eine oder mehrere Verbindungen der folgenden Formel enthält: 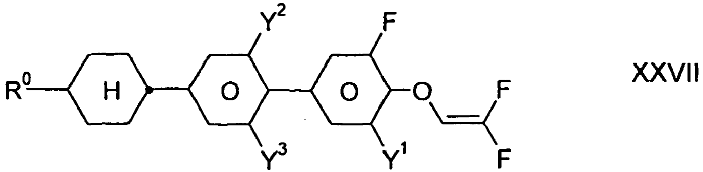 worin R⁰ und Y¹⁻³ die oben angegebenen Bedeutungen besitzen. Besonders bevorzugt sind Verbindungen der folgenden Formeln: 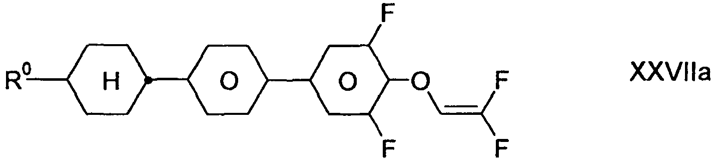 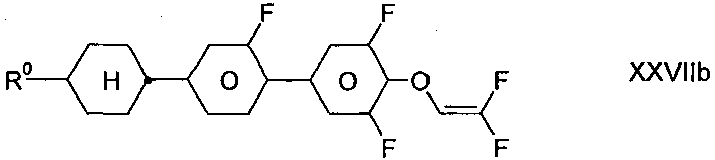 worin R⁰ die oben angegebene Bedeutung besitzt und vorzugsweise Alkyl, Alkoxy, Oxaalkyl, Fluoralkyl oder Alkenyl mit jeweils bis zu 8 C-Atomen bedeutet.
- FK-Medium, welches zusätzlich eine oder mehrere Verbindungen der folgenden Formel enthält: 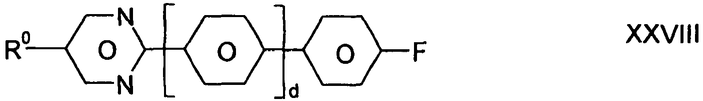 worin R⁰ die oben angegebene Bedeutung besitzt und vorzugsweise geradkettiges Alkyl mit 2-5 C-Atomen ist, und d 0 oder 1, vorzugsweise 1 bedeutet. Bevorzugte Mischungen enthalten 3 - 30 Gew.%, insbesondere 5 - 20 Gew.% dieser Verbindung(en).
- FK-Medium, welches zusätzlich eine oder mehrere Verbindungen der folgenden Formel enthält: 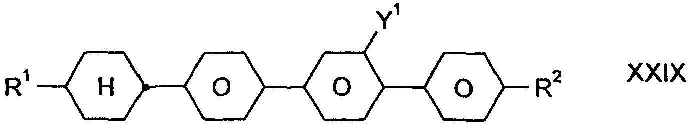 worin Y¹, R¹ und R² die oben angegebene Bedeutung besitzen. R¹ und R² bedeuten vorzugsweise jeweils unabhängig voneinander Alkyl mit 1 bis 8 C-Atomen. Y¹ bedeutet vorzugsweise F. Bevorzugte Mischungen enthalten 1 - 15 Gew.%, insbesondere 1 - 10 Gew.% dieser Verbindungen. 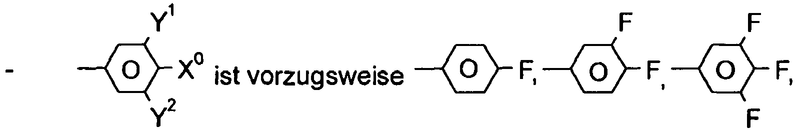 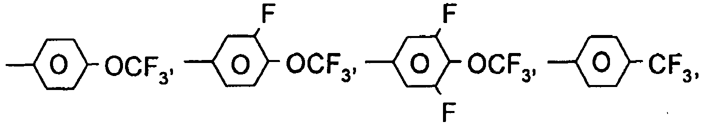 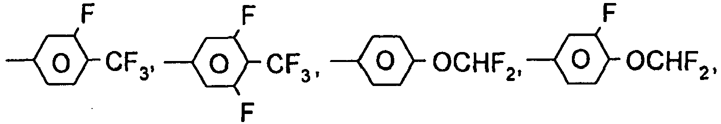 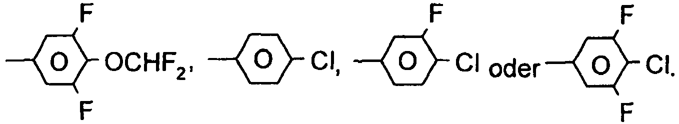
- R⁰ ist vorzugsweise geradkettiges Alkyl oder Alkenyl mit 2 bis 7 C-Atomen;
- X⁰ ist vorzugsweise F, ferner OCF₃, Cl oder CF₃;
- Das Medium enthält vorzugsweise eine, zwei oder drei Verbindungen der Formel I;
- Das Medium enthält vorzugsweise eine oder mehrere Verbindungen ausgewählt aus der Gruppe der Verbindungen der Formeln II, III, VI-2, XI, XII, XIII, XIV, XXIV, XXV, XXVI, XXVII und XXIX.
- Das Medium enthält vorzugsweise jeweils eine oder mehrere Verbindungen der Formel VI-2, XI und XXVI;
- Das Medium enthält vorzugsweise 3-80 Gew.%, bevorzugt 3-50 Gew.% an Verbindungen der Formel I;
- Der Anteil an Verbindungen der Formeln II-XXVIII im Gesamtgemisch beträgt vorzugsweise 20 bis 99 Gew.%;
- Das Medium enthält vorzugsweise 25-80 Gew.%, besonders bevorzugt 30-70 Gew.% an Verbindungen der Formel II und/oder III;
- Das Medium enthält vorzugsweise 20-70 Gew.%, besonders bevorzugt 25-60 Gew.% an Verbindungen der Formel IIa, insbesondere worin R^{3a} H bedeutet;
- Das Medium enthält vorzugsweise 2-20 Gew.%, besonders bevorzugt 3-15 Gew.% an Verbindungen der Formel VI-2;
- Das Medium enthält 2-20 Gew.%, besonders bevorzugt 3-15 Gew.% an Verbindungen der Formel XI;
- Das Medium enthält vorzugsweise 1-20 Gew.%, besonders bevorzugt 2-15 Gew.% an Verbindungen der Formel XXIV;
- Das Medium enthält vorzugsweise 1-25 Gew.%, besonders bevorzugt 2-20 Gew.% an Verbindungen der Formel XXVI;
- Das Medium enthält vorzugsweise 1-35 Gew.%, besonders bevorzugt 5-30 Gew.% an Verbindungen der Formel XXVII;
- Das Medium enthält vorzugsweise eine oder mehrere Verbindungen ausgewählt aus der Gruppe der Verbindungen der Formeln VI-2, VII-1a, VII-1b, IX, X, XI und XXVI (CF₂O-verbrückte Verbindungen).

Es wurde gefunden, dass bereits ein relativ geringer Anteil an Verbindungen der Formel S1 im Gemisch mit üblichen Flüssigkristallmaterialien, insbesondere jedoch mit einer oder mehreren Verbindungen der Formel S2 zu einer beträchtlichen Erhöhung der Lichtstabilität führt, wobei gleichzeitig breite nematische Phasen mit tiefen Übergangstemperaturen smektisch-nematisch beobachtet werden, wodurch die Lagerstabilität verbessert wird. Gleichzeitig zeigen die Mischungen sehr geringe Schaltzeiten und sehr gute Werte für die VHR nach UV-Belastung und bei erhöhter Temperatur.

Messungen des "Voltage Holding-ratio" (HR) [S. Matsumoto et al., Liquid Crystals 5, 1320 (1989); K. Niwa et al., Proc. SID Conference, San Francisco, June 1984, p. 304 (1984); G. Weber et al., Liquid Crystals 5, 1381 (1989)] haben ergeben, dass erfindungsgemäße Mischungen enthaltend Verbindungen der Formel I eine deutlich geringere Abnahme des HR unter UV-Belastung aufweisen als analoge Mischungen enthaltend anstelle den Verbindungen der Formel I Cyanophenylcyclohexane der Formel 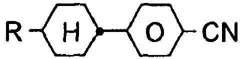 oder Ester der Formel 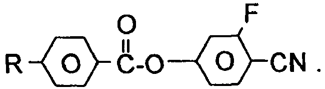 Die LC-Medien sind vorzugsweise zu 99 Gew.%, besonders bevorzugt zu 100 Gew.% frei von Benzonitrilderivaten.

Die Lichtstabilität und UV-Stabilität der erfindungsgemäßen Mischungen ist erheblich besser, d.h. sie zeigen eine deutlich kleinere Abnahme des HR unter Licht- bzw. UV-Belastung als unstabilisierte Mischungen. Bereits geringe Konzentrationen der Verbindungen (≤ 1 Gew.%) der Formeln S1 und/oder S2 in den Mischungen erhöhen die HR gegenüber Mischungen aus dem Stand der Technik um 10 % und mehr.

Der Ausdruck "Alkyl" bzw. "Alkyl*" umfasst in dieser Anmeldung geradkettige und verzweigte Alkylgruppen mit 1-7 Kohlenstoffatomen, insbesondere die geradkettigen Gruppen Methyl, Ethyl, Propyl, Butyl, Pentyl, Hexyl und Heptyl. Gruppen mit 1-6 Kohlenstoffatomen sind im allgemeinen bevorzugt.

Der Ausdruck "Alkenyl" bzw. "Alkenyl*" umfasst in dieser Anmeldung geradkettige und verzweigte Alkenylgruppen mit 2-7 Kohlenstoffatomen,insbesondere die geradkettigen Gruppen. Bevorzugte Alkenyl-gruppen sind C₂-C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1 E-Alkenyl, C₄-C₇-3E-Alkenyl und C₅-C₇-4-Alkenyl. Beispiele besonders bevorzugter Alkenyl-gruppen sind Vinyl, 1 E-Propenyl, 1 E-Butenyl, 1 E-Pentenyl, 1 E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfasst in dieser Anmeldung geradkettige Gruppen mit mindestens einem Fluoratom, vorzugsweise einem endständigem Fluor, d.h. Fluormethyl, 2-Fluorethyl, 3-Fluorpropyl, 4-Fluor-butyl, 5-Fluorpentyl, 6-Fluorhexyl und 7-Fluorheptyl. Andere Positionen des Fluors sind jedoch nicht ausgeschlossen.

Der Ausdruck "Oxaalkyl" bzw. "Alkoxy" umfasst in dieser Anmeldung geradkettige Reste der Formel CₙH₂ₙ₊₁-O-(CH₂)ₘ, worin n und m jeweils unabhängig voneinander 1 bis 6 bedeuten. m kann auch 0 bedeuten. Vorzugsweise ist n = 1 und m 1-6 oder m = 0 und n = 1-3.

Der Ausdruck "halogenierter Alkylrest" umfasst vorzugsweise ein- oder mehrfach fluorierte und/oder chlorierte Reste. Perhalogenierte Reste sind eingeschlossen. Besonders bevorzugt sind fluorierte Alkylreste, insbesondere CF₃, CH₂CF₃, CH₂CHF₂, CHF₂, CH₂F, CHFCF₃ und CF₂CHFCF₃.

Falls in den oben- und untenstehenden Formeln R⁰ einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradedoxy.

Oxaalkyl bedeutet vorzugsweise geradkettiges 2-Oxapropyl (= Methoxymethyl), 2-(= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6-, oder 7-Oxaoctyl, 2-, 3-, 4-, 5-, 6-, 7- oder 8-Oxanonyl, 2-, 3-, 4-, 5-, 6-, 7-, 8- oder 9-Oxadexyl.

Falls R⁰ einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl. Diese Reste können auch ein- oder mehrfach halogeniert sein.

Falls R⁰ einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

In den oben- und untenstehenden Formeln ist X⁰ vorzugsweise F, Cl oder ein ein- oder mehrfach fluorierter Alkyl- oder Alkoxyrest mit 1, 2 oder 3 C-Atomen oder ein ein- oder mehrfach fluorierter Alkenylrest mit 2 oder 3 C-Atomen. X⁰ ist besonders bevorzugt F, Cl, CF₃, CHF₂, OCF₃, OCHF₂, OCFHCF₃, OCFHCHF₂, OCFHCHF₂, OCF₂CH₃, OCF₂CHF₂, OCF₂CHF₂, OCF₂CF₂CHF₂, OCF₂CF₂CH₂F, OCFHCF₂CF₃, OCFHCF₂CHF₂, OCH=CF₂, OCF=CF₂, OCF₂CHFCF₃, OCF₂CF₂CF₃, OCF₂CF₂CCIF₂, OCCIFCF₂CF₃, CF=CF₂, CF=CHF, oder CH=CF₂, ganz besonders bevorzugt F oder OCF₃.

Das optimale Mengenverhältnis der Verbindungen der oben genannten Formeln hängt weitgehend von den gewünschten Eigenschaften, von der Wahl der Komponenten der oben genannten Formeln und der Wahl weiterer gegebenenfalls vorhandener Komponenten ab.

Geeignete Mengenverhältnisse innerhalb des oben angegebenen Bereichs können von Fall zu Fall leicht ermittelt werden.

Die einzelnen Verbindungen der oben genannten Formeln und deren Unterformeln, die in den erfindungsgemäßen Medien verwendet werden können, sind entweder bekannt, oder sie können analog zu den bekannten Verbindungen hergestellt werden.

Gegenstand der Erfindung sind auch elektrooptische Anzeigen, wie z. B. TN-, VA-, TFT-, OCB-, IPS-, FFS- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit vorzugsweise hohem spezifischem Widerstand, die derartige Medien enthalten sowie die Verwendung dieser Medien für elektrooptische Zwecke.

Die erfindungsgemäßen Flüssigkristallmischungen ermöglichen eine bedeutende Erweiterung des zur Verfügung stehenden Parameterraumes. Die erzielbaren Kombinationen aus Klärpunkt, Viskosität bei tiefer Temperatur, thermischer und UV-Stabilität und hoher optischer Anisotropie übertreffen bei weitem bisherige Materialien aus dem Stand der Technik.

Die erfindungsgemäßen Mischungen sind insbesondere für mobile Anwendungen und high-Δn-TFT-Anwendungen wie z. B. PDAs, Notebooks, LCD-TV und Monitore geeignet.

Die erfindungsgemäßen Flüssigkristallmischungen ermöglichen es, bei Beibehaltung der nematischen Phase bis -20 °C und bevorzugt bis -30 °C, besonders bevorzugt bis -40 °C, und des Klärpunkts ≥ 70 °C, vorzugsweise ≥ 75 °C, gleichzeitig Rotationsviskositäten γ₁ von≤ 100 mPa·s, besonders bevorzugt≤ 70 mPa·s zu erreichen, wodurch hervorragende MFK-Anzeigen mit schnellen Schaltzeiten erzielt werden können.

Die dielektrische Anisotropie der erfindungsgemäßen Flüssigkristallmischungen Λε ist vorzugsweise ≥ +5, besonders bevorzugt ≥ +9. Die Mischungen sind außerdem durch kleine Operationsspannungen gekennzeichnet. Die Schwellenspannung der erfindungsgemäßen Flüssigkristallmischungen ist vorzugsweise ≤ 1,7 V, insbesondere ≤ 1,6 V.

Die Doppelbrechung Δn der erfindungsgemäßen Flüssigkristallmischungen ist vorzugsweise ≥ 0,12, besonders bevorzugt ≥ 0,13.

Der nematische Phasenbereich der erfindungsgemäßen Flüssigkristallmischungen ist vorzugsweise mindestens 90°, insbesondere mindestens 100° breit. Vorzugsweise erstreckt sich dieser Bereich mindestens von -25 °C bis +70 °C.

Es versteht sich, dass durch geeignete Wahl der Komponenten der erfindungsgemäßen Mischungen auch höhere Klärpunkte (z.B. oberhalb 100 °C) bei höheren Schwellenspannungen oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringen Schwellen erhalten werden.

Die FK-Medien können auch weitere, dem Fachmann bekannte und in der Literatur beschriebene Zusätze, wie z. B. UV-Stabilisatoren wie Tinuvin^{®} der Fa. Ciba, Antioxidantien, Radikalfänger, Nanopartikel, etc. enthalten. Beispielsweise können 0-15 % pleochroitische Farbstoffe oder chirale Dotierstoffe zugesetzt werden. Geeignete Stabilisatoren und Dotierstoffe werden nachfolgend in den Tabellen C und D genannt.

Die einzelnen Komponenten der oben genannten bevorzugten Ausführungsformen der erfindungsgemäßen FK-Medien sind entweder bekannt, oder ihre Herstellungsweisen sind für den einschlägigen Fachmann aus dem Stand der Technik ohne weiteres abzuleiten, da sie auf in der Literatur beschriebenen Standardverfahren basieren.

Es versteht sich für den Fachmann von selbst, daß die erfindungsgemäßen FK-Medien auch Verbindungen enthalten können, worin beispielsweise H, N, O, Cl, F durch die entsprechenden Isotope ersetzt sind.

Die Herstellung der erfindungsgemäß verwendbaren Flüssigkristallmischungen erfolgt in an sich üblicher Weise, beispielsweise indem man eine oder mehrere Verbindungen der Formeln I, S1 und optional S2 mit einer oder mehreren Verbindungen der Formeln II-XXVIII oder mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z. B. in Aceton, Chloroform oder Methanol, zu mischen und das Lösungsmittel nach Durchmischung wieder zu entfernen, beispielsweise durch Destillation. Das Verfahren zur Herstellung der erfindungsgemäßen FK-Medien ist ein weiterer Gegenstand der Erfindung.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die folgenden Beispiele erläutern die vorliegende Erfindung ohne sie zu begrenzen. Sie zeigen dem Fachmann jedoch bevorzugte Mischungskonzepte mit bevorzugt einzusetzenden Verbindungen und deren jeweiligen Konzentrationen sowie deren Kombinationen miteinander. Außerdem illustrieren die Beispiele, welche Eigenschaften und Eigenschftskombinationen zugänglich sind.

In der vorliegenden Anmeldung und in den folgenden Beispielen sind die Strukturen der Flüssigkristallverbindungen durch Akronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen; n, m und k sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Akronym für den Grundkörper angegeben. Im Einzelfall folgt getrennt von Akronym für den Grundkörper mit einem Strich ein Code für die Substituenten R^{1*}, R^{2*}, L^{1*} und L^{2*}:

| Code für R^{1*}, R^{2*}, L^{1*}, L^{2*}, L^{3*} | R^{1*} | R^{2*} | L^{1*} | L^{2*} |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | F | H |
| nN.F.F | CₙH₂ₙ₊₁ | CN | F | F |
| nF | CₙH₂ₙ₊₁ | F | H | H |
| nCl | CₙH₂ₙ₊₁ | Cl | H | H |
| nOF | OCₙH₂ₙ₊₁ | F | H | H |
| nF.F | CₙH₂ₙ₊₁ | F | F | H |
| nF.F.F | CₙH₂ₙ₊₁ | F | F | F |
| nOCF₃ | CₙH₂ₙ₊₁ | OCF₃ | H | H |
| nOCF₃.F | CnH2n+1 | OCF₃ | F | H |
| n-Vm | CₙH₂ₙ₊₁ | -CH=CH-CₘH₂ₘ₊₁ | H | H |
| nV-Vm | CₙH₂ₙ₊₁-CH=CH- | -CH=CH-CₘH₂ₘ₊₁ | H | H |

Bevorzugte Mischungskomponenten finden sich in den Tabellen A und B.

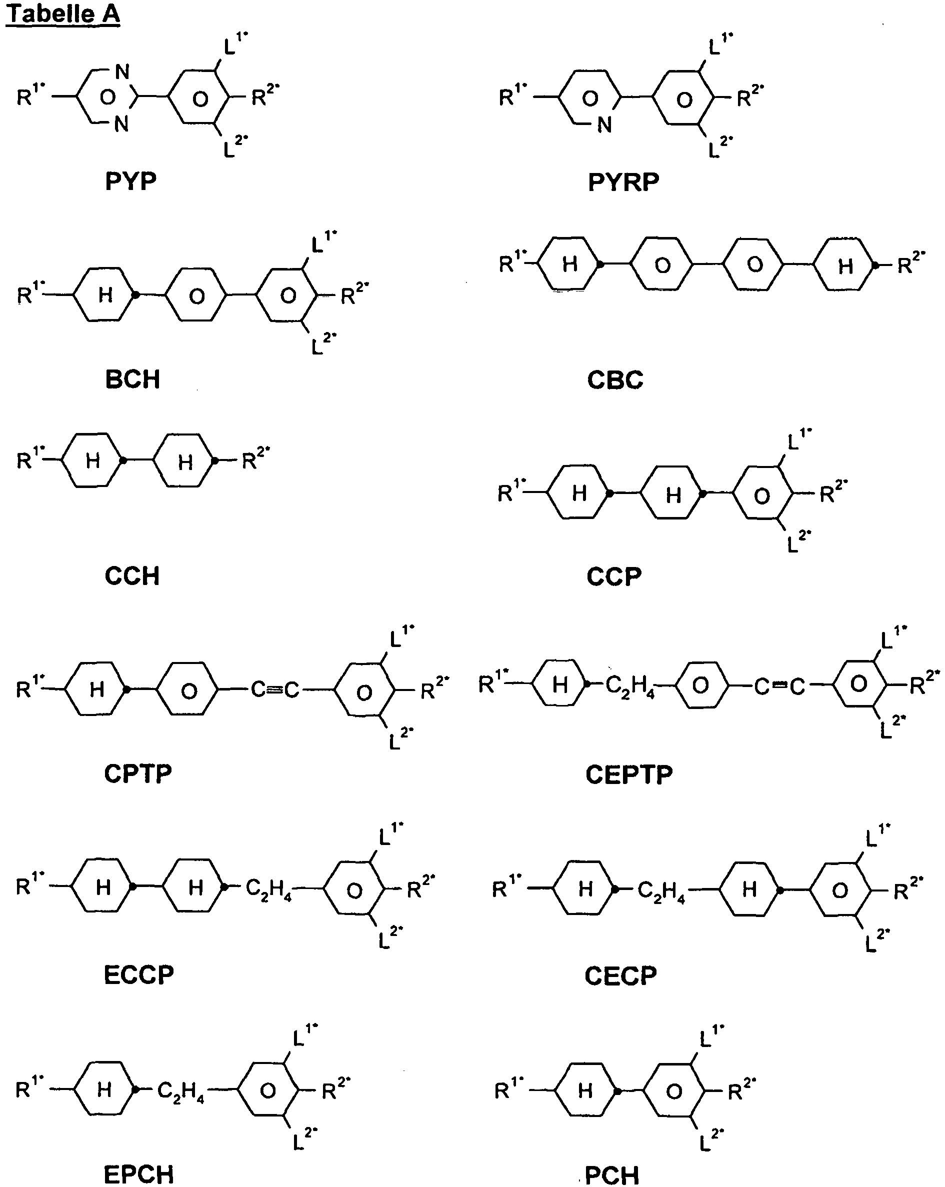

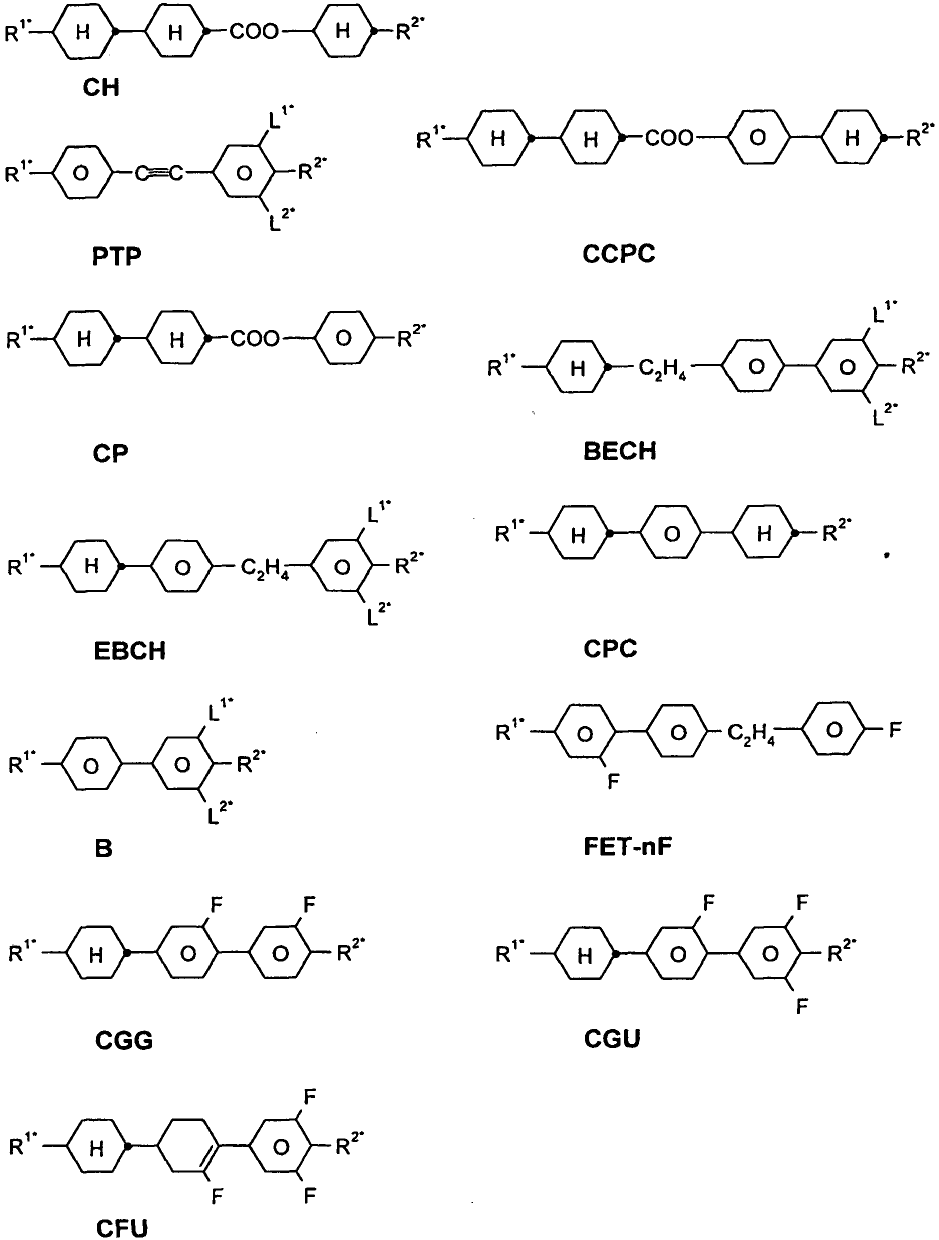

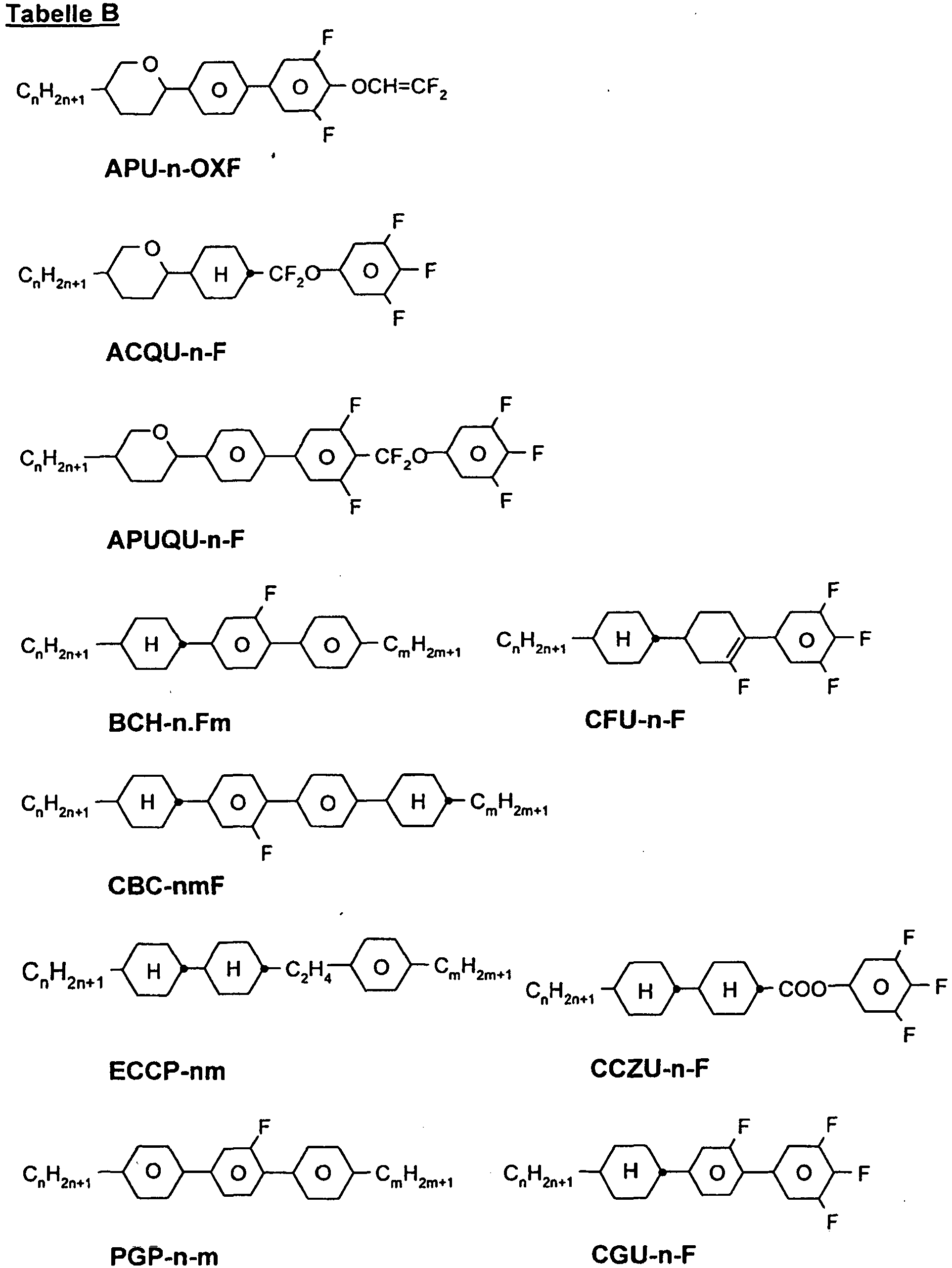

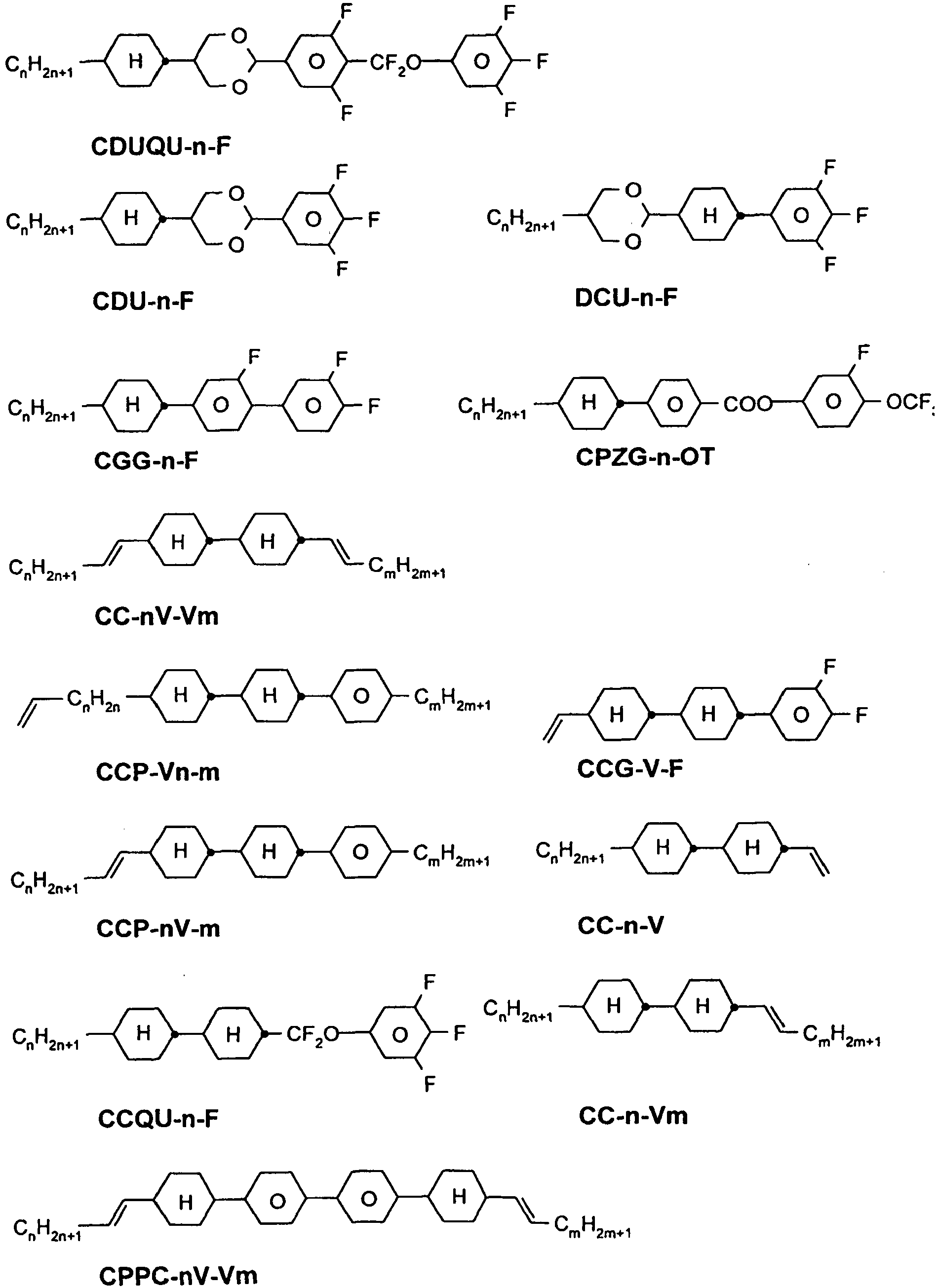

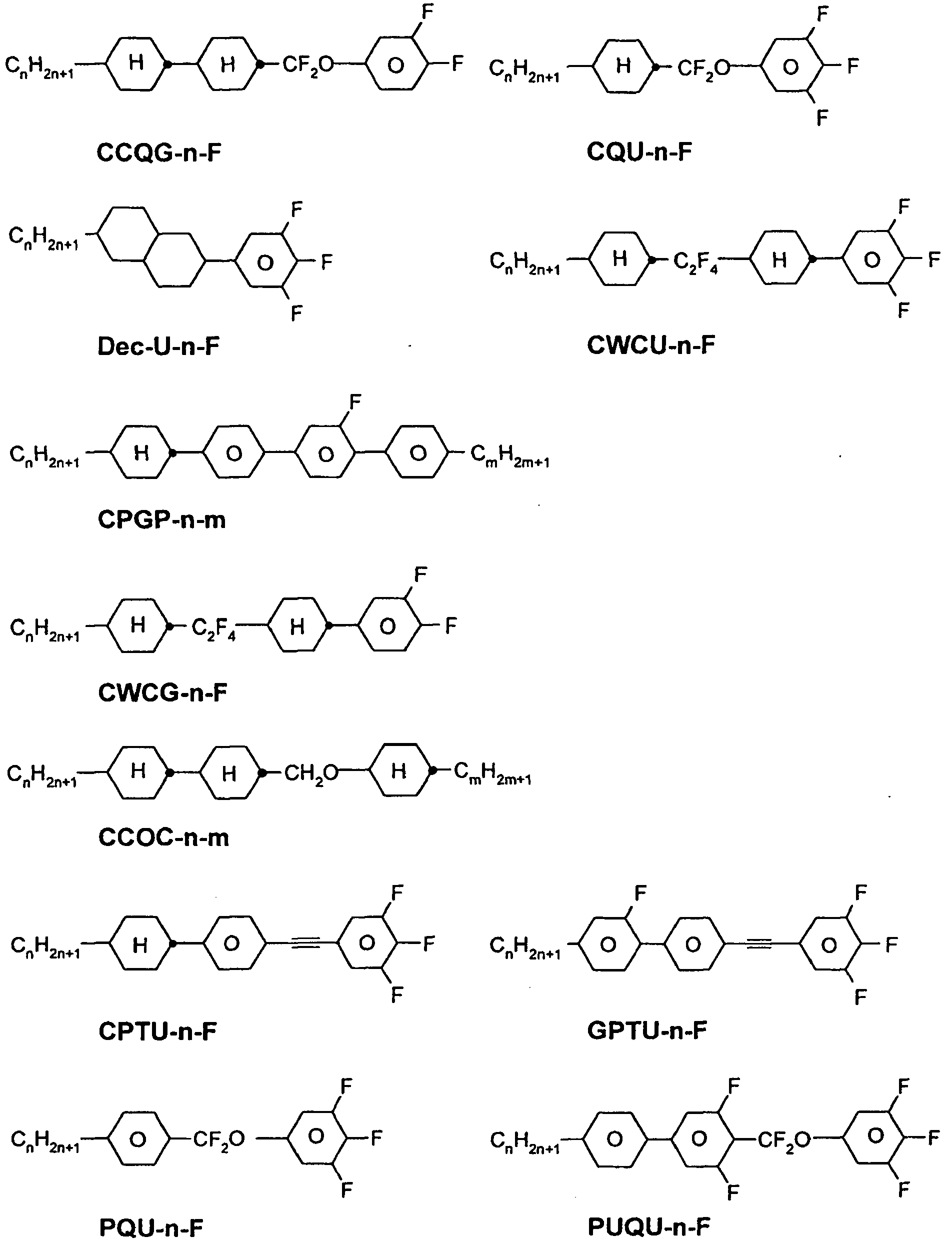

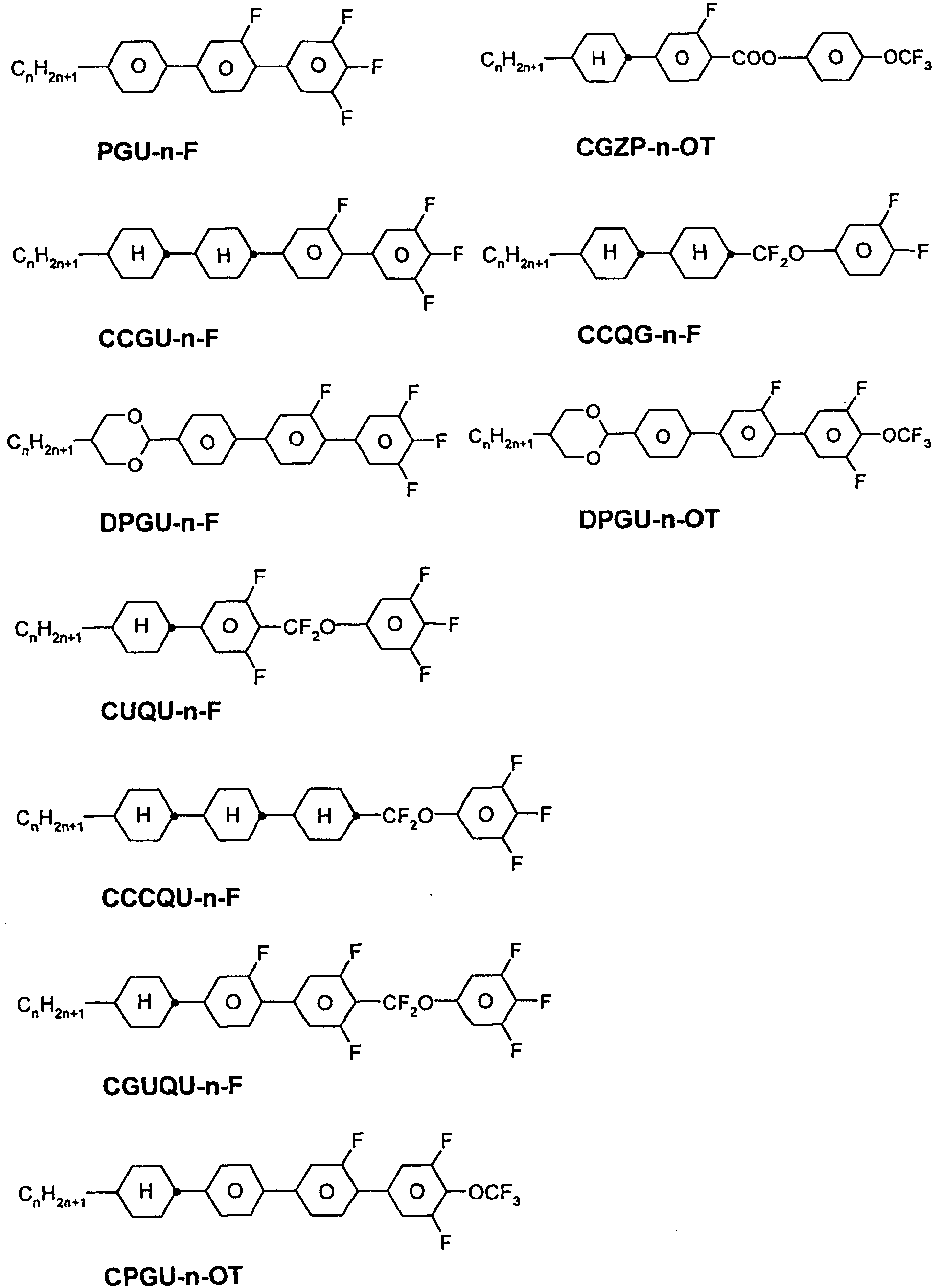

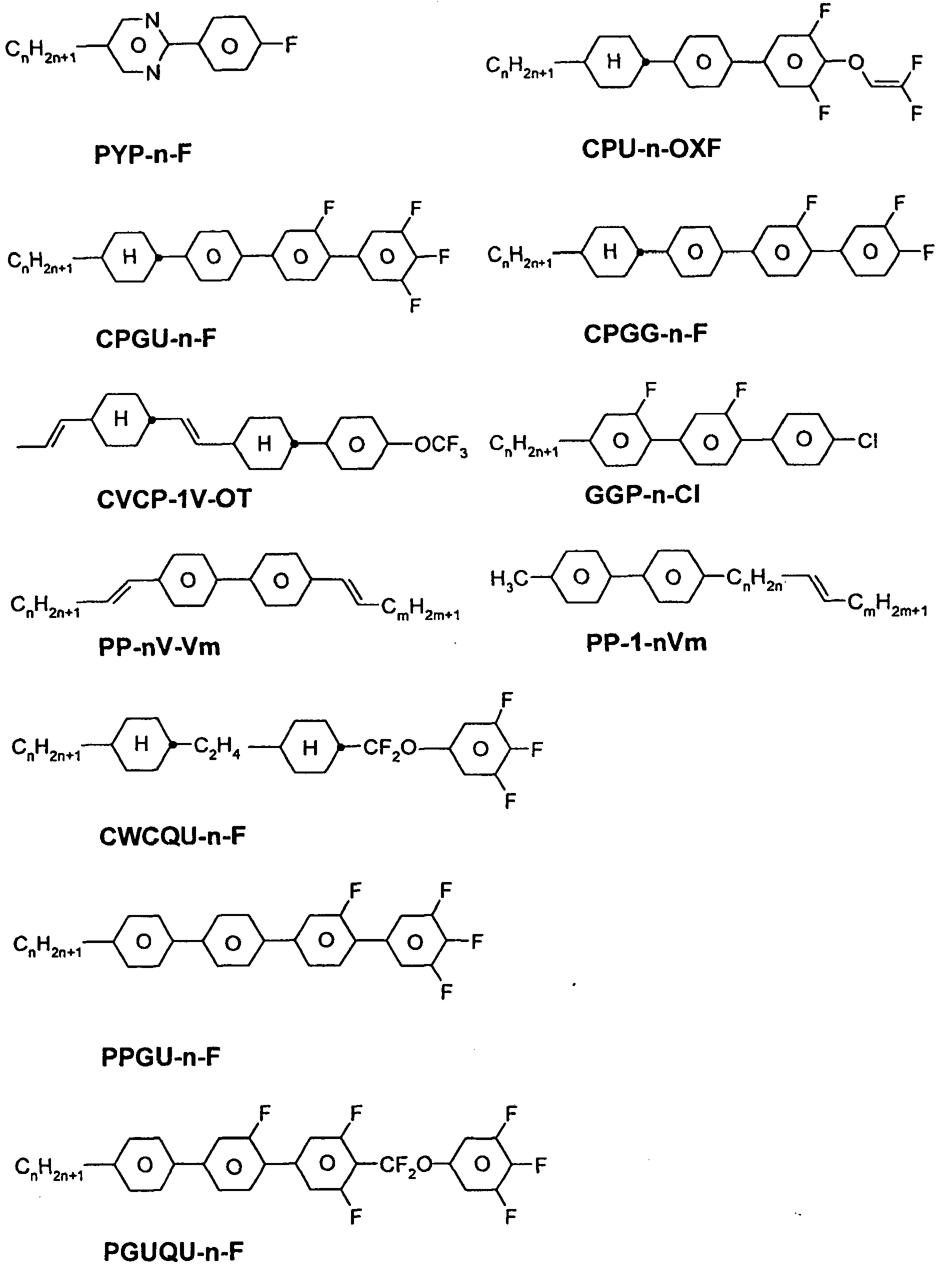

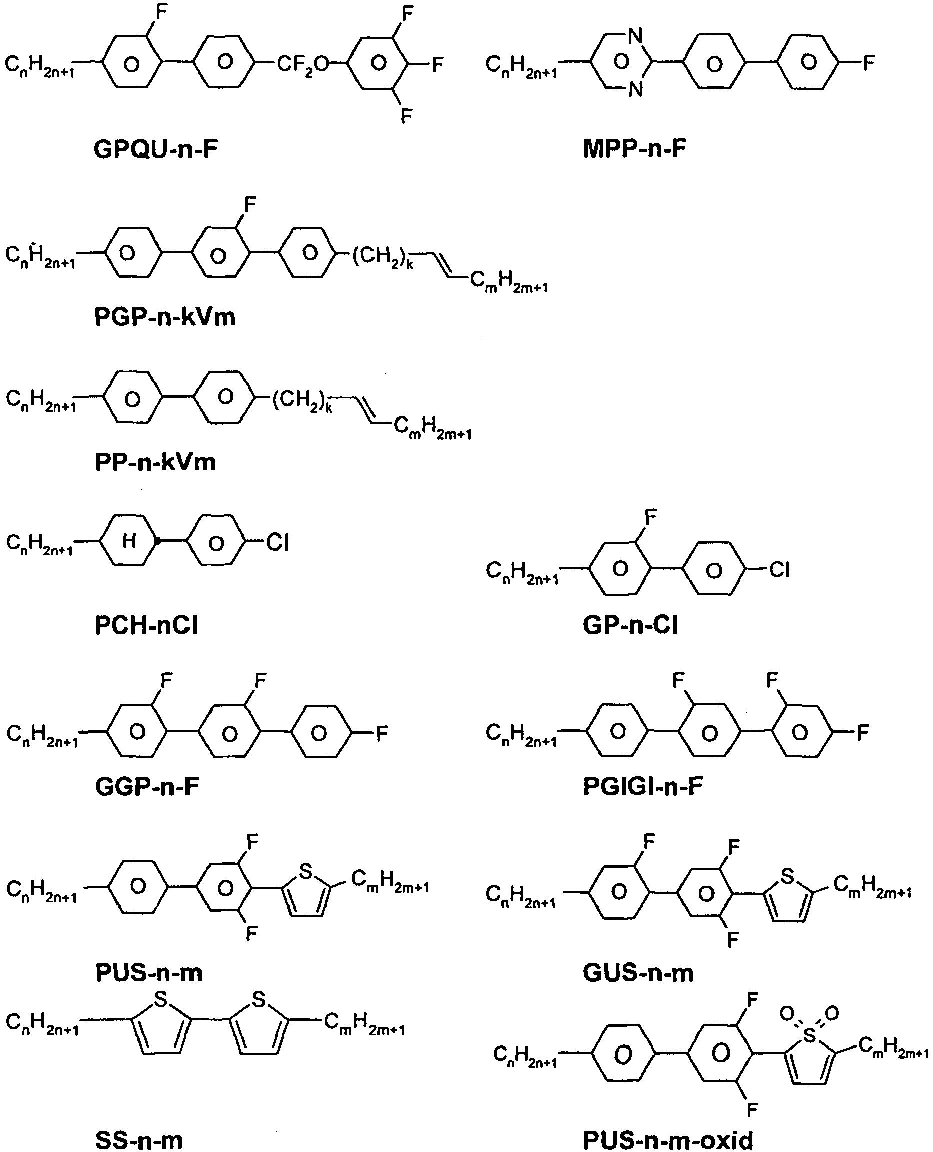

In einer bevorzugten Ausführungsform der vorliegenden Erfindung enthalten die erfindungsgemäßen FK-Medien eine oder mehrere Verbindungen ausgewählt aus der Gruppe bestehend aus Verbindungen der Tabelle A und B.

**Tabelle C**

| In der Tabelle C werden mögliche Dotierstoffe angegeben, die den erfindungsgemäßen FK-Medien zugesetzt werden können. | |
|---|---|
| 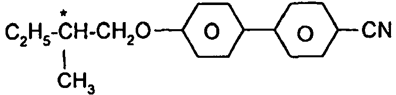 | 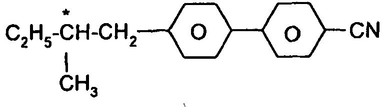 |
| **C15** | **CB 15** |
| 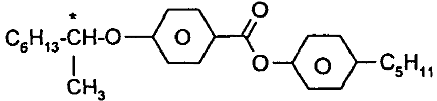 | 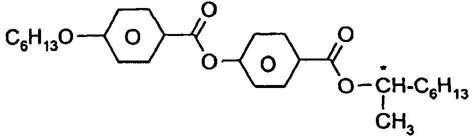 |
| **CM 21** | **R/S-811** |
| 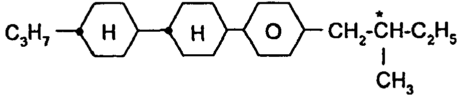 | 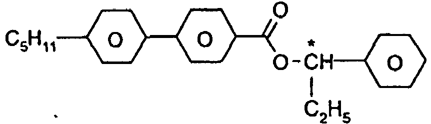 |
| **CM 44** | **CM 45** |
| 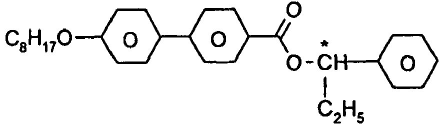 | 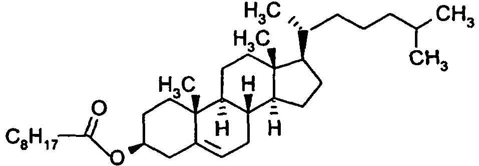 |
| **CM 47** | **CN** |
| 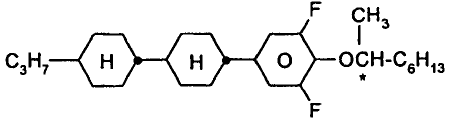 | 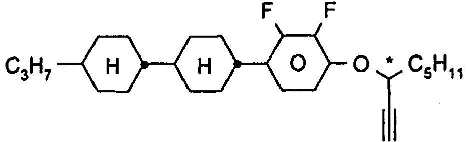 |
| **R/S-2011** | **R/S-3011** |
| 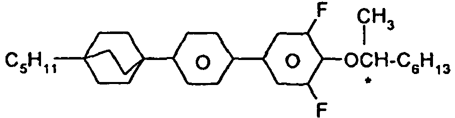 | 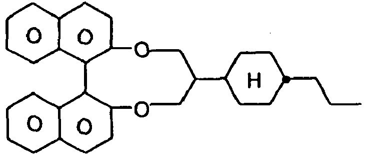 |
| **R/S-4011** | **R/S-5011** |
| 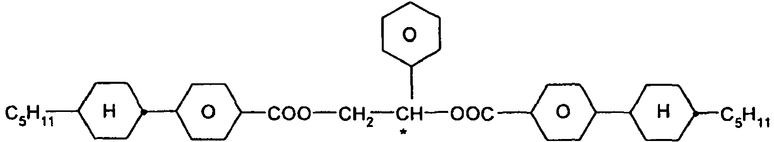 | |
| **R/S-1011** | |

Vorzugsweise enthalten die FK-Medien 0 bis 10 Gew.%, insbesondere 0,01 bis 5 Gew.% und besonders bevorzugt 0,1 bis 3 Gew.% an Dotierstoffen. Vorzugsweise enthalten die FK-Medien einen oder mehrere Dotierstoffe ausgewählt aus der Guppe bestehend aus Verbindungen der Tabelle C.

**Tabelle D**

| In der Tabelle D werden weitere mögliche Stabilisatoren angegeben, die den erfindungsgemäßen FK-Medien neben den Verbindungen S1 und S2 zugesetzt werden können. | |
|---|---|
| (n bedeutet hier eine ganze Zahl von 1 bis 12) | |
| 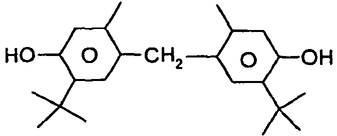 | 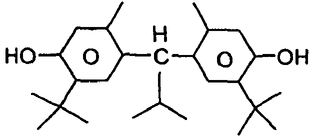 |
| 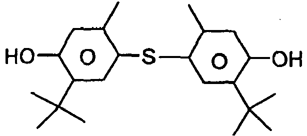 | 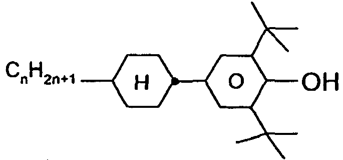 |
| 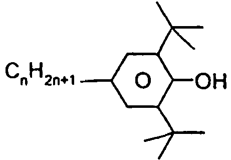 | 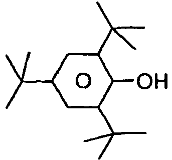 |
| 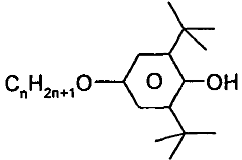 | 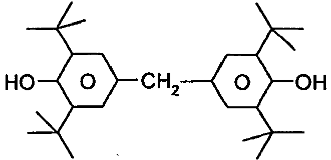 |
| 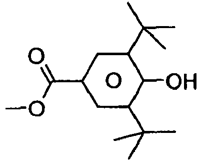 | 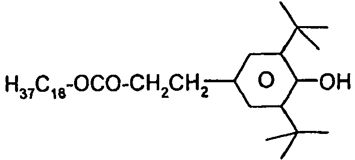 |
| 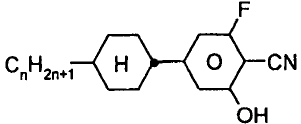 | 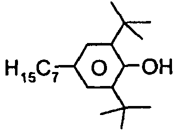 |
| 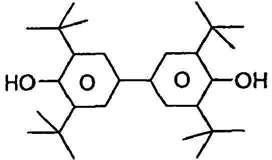 | 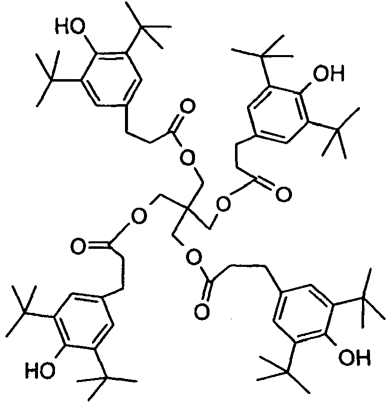 |
| 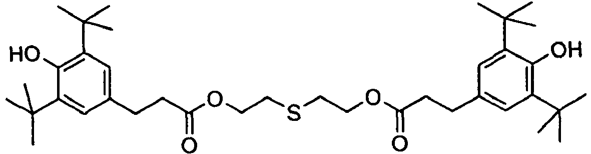 | |
| 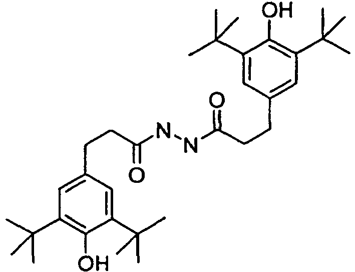 | 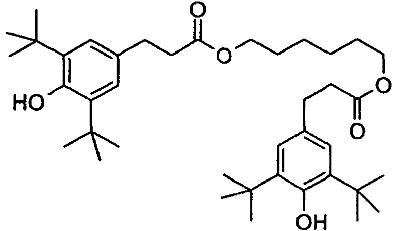 |
| 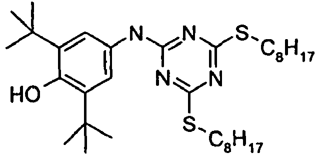 | 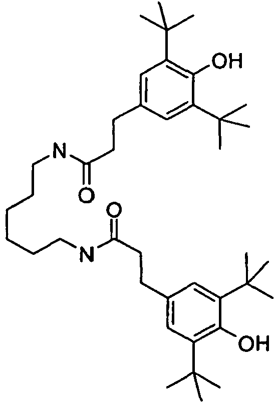 |
| 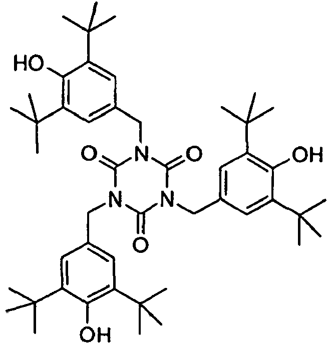 | 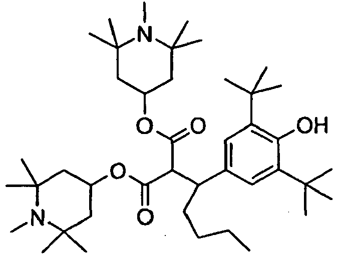 |
| 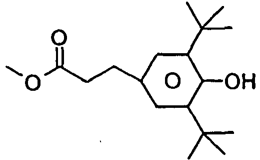 | 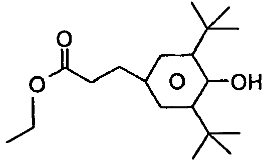 |
| 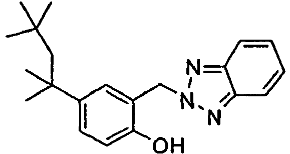 | 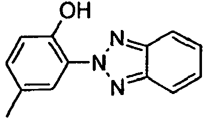 |
| 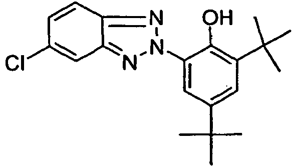 | 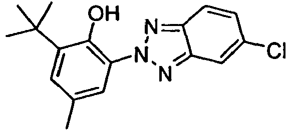 |
| 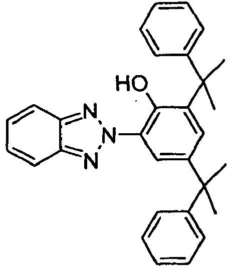 | 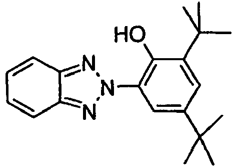 |
| 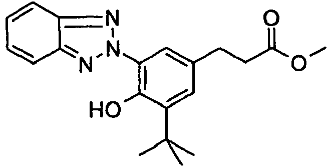 | 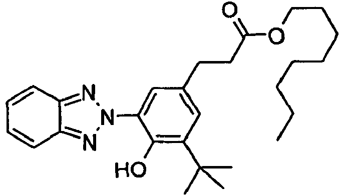 |
| 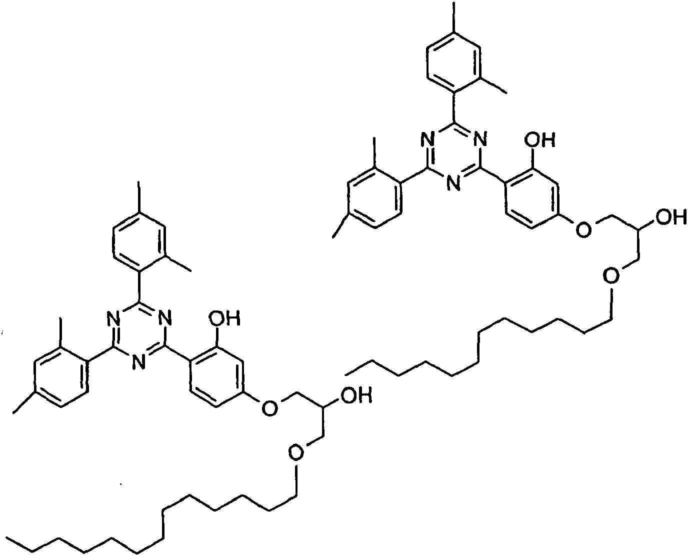 | |
| 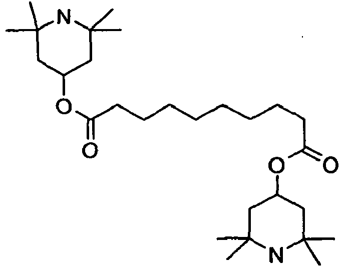 | 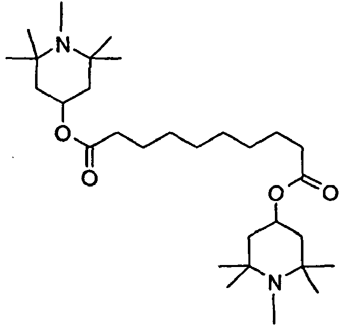 |

Vorzugsweise enthalten die FK-Medien 0 bis 10 Gew.%, insbesondere 0,01 bis 5 Gew.% und besonders bevorzugt 0,1 bis 3 Gew.% an Stabilisatoren. Vorzugsweise enthalten die FK-Medien einen oder mehrere Stabilisatoren ausgewählt aus der Gruppe bestehend aus Verbindungen der Tabelle D.

Außerdem werden folgende Abkürzungen und Symbole verwendet:
- Vₒ: Schwellenspannung, kapazitiv [V] bei 20°C,
- V₁₀: optische Schwelle für 10 % relativen Kontrast [V] bei 20°C,
- nₑ: außerordentlicher Brechungsindex bei 20°C und 589 nm,
- nₒ: ordentlicher Brechungsindex bei 20°C und 589 nm,
- Δn: optische Anisotropie bei 20°C und 589 nm,
- ε_{⊥}: dielektrische Suszeptibilität senkrecht zum Direktor bei 20°C und 1 kHz,
- ε∥: dielektrische Suszeptibilität parallel zum Direktor bei 20°C und 1 kHz,
- Δε: dielektrische Anisotropie bei 20°C und 1 kHz,
- Kp., T(N,I): Klärpunkt [°C],
- γ1: Rotationsviskosität bei 20°C [mPa·s],
- K₁: elastische Konstante, "splay"-Deformation bei 20°C [pN],
- K₂: elastische Konstante, "twist"-Deformation bei 20°C [pN],
- K₃: elastische Konstante, "bend"-Deformation bei 20°C [pN],
- LTS: "low temperature stability" (Phase), bestimmt in Testzellen,
- tₒₙ: Einschaltzeit [Transmissionsänderung von 10 % auf 90 %) bei 20 °C und definierter Operationsspannung,
- t_{off}: Ausschaltzeit [Transmissionsänderung von 90 % auf 10 %) bei 20 °C,
- RT: Reaktionszeit (tₒₙ + t_{off})
- HR₂₀: "voltage holding ratio" bei 20°C [%] und
- HR₁₀₀: "voltage holding ratio" bei 100°C [%].

Soweit nicht explizit anders vermerkt, sind in der vorliegenden Anmeldung alle Konzentrationen in Gewichtsprozent angegeben und beziehen sich auf die entsprechende Gesamtmischung ohne Lösungsmittel.

Soweit nicht explizit anders vermerkt, sind in der vorliegenden Anmeldung alle angegebenen Werte für Temperaturen, wie z. B. der Schmelzpunkt T(C,N), der Übergang von der smektischen (S) zur nematischen (N) Phase T(S,N) und der Klärpunkt T(N,I), in Grad Celsius (°C) angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar.

Alle physikalischen Eigenschaften werden und wurden nach "Merck Liquid Crystals, Physical Properties of Liquid Crystals", Status Nov. 1997, Merck KGaA, Darmstadt, Deutschland bestimmt und gelten für eine Temperatur von 20°C und An wird bei 589 nm und Δε bei 1 kHz bestimmt, sofern nicht jeweils explizit anders angegeben.

Die flüssigkristallinen Eigenschaften der Einzelverbindungen werden, soweit nicht anders angegeben, in der nematischen Wirtsmischung ZLI-4792 (kommerziell erhältlich von Merck KGaA, Darmstadt) bei einer Konzentration von 10% bestimmt.

"Raumtemperatur" bedeutet 20°C, falls nicht anders angegeben.

Der Begriff "Schwellenspannung" bezieht sich für die vorliegende Erfindung auf die kapazitive Schwelle (V₀), auch Freedericksz-Schwelle genannt, sofern nicht explizit anders angegeben. In den Beispielen kann auch, wie allgemein üblich, die optische Schwelle für 10 % relativen Kontrast (V₁₀) angegeben werden.

Die zur Messung der kapazitiven Schwellenspannung Vₒ sowie für V₁₀ verwendeten Testzellen sind aufgebaut aus Substraten bestehend aus Natriumglas (Sodalime Glas), die mit Polyimidorientierungsschichten (Durimid® 32 mit Verdünner (70 % NMP + 30 % Xylol) im Verhältnis 1:4) der Firma Arch Chemicals beschichtet sind, welche antiparallel zueinander gerieben sind und einen Oberflächentilt von quasi 0 Grad aufweisen. Die Fläche der durchsichtigen, nahezu quadratischen Elektroden aus ITO, beträgt 1 cm². Zur Bestimmung der kapazitiven Schwellenspannung wird ein handelsübliches hochauflösendes LCR-Meter (z.B. LCR-Meter 4284A der Firma Hewlett Packard) eingesetzt.

### Beispiele

Die Dithiophenverbindung SS-2-2 wird nach Literaturvorschrift (Kumada, M. et al., K. Org. Synth. Coll. Vol. 6 1988, 407-411; Engelmann, G. et al. J. Chem. Soc., Perkin Trans. 2 1998, 169-175 und enthaltene Zitate) hergestellt.

### Beispiel 1

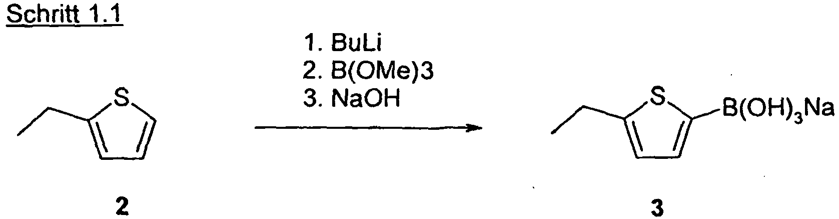

Unter Stickstoff wird eine Lösung von 116g (950 mmol) 2-Ethylthiophen in 200 ml THF bei -70°C mit 656 ml (15%ige Lösung in *n*-Hexan) (1.04 mol) Butyllithium versetzt. Anschließend wird der Ansatz 30 min bei -70°C und 20 min bei -20°C gerührt. Bei -70°C wird dem Gemisch 124 ml (1.09 mol) Trimethylborat zugesetzt und 30 min bei tiefer Temperatur gerührt. Das Kältebad wird entfernt, und der Ansatz wird bei -15°C mit 500 ml Wasser verdünnt und mit Salzsäure angesäuert. Die wässrige Phase wird mit MTB-Ether extrahiert, die vereinigten organischen Phasen werden mit ges. Natriumchloridlösung gewaschen, über Natriumsulfat getrocknet und eingeengt. Der Rückstand wird in 1 l THF gelöst und unter Rühren und Kühlen mit 80 ml 50%iger Natronlauge versetzt. Der Ansatz wird auf -10°C abgekühlt und der ausgefallene Feststoff abgetrennt. Ausbeute 89 %.

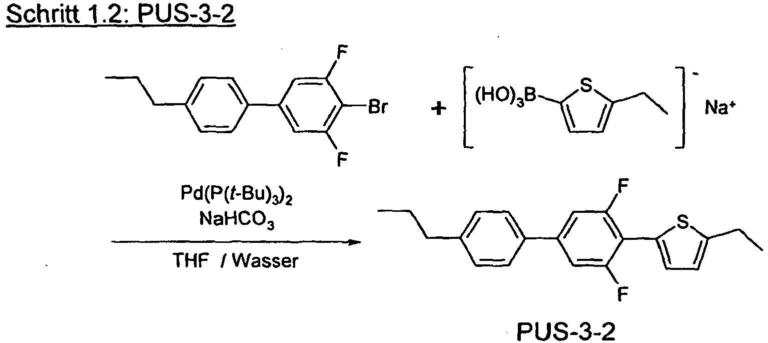

17,0 g (54,6 mmol) 4-Brom-3,5-difluor-4'-propyl-biphenyl, 16,1 g (82,1 mmol) 2-Ethylthiophen-5-hydroxyboronsäurenatriumsalz und 9,2 g (0,11 mol) Natriumhydrogencarbonat werden zusammen mit 700 mg (1,37 mmol) Bis(tri-*tert-*butylphosphin)palladium(0) in 250 ml THF-WasserGemisch (2:1) refluxiert. Nach 22 h wird die Mischung mit MTBE verdünnt, und die organische Phase wird abgetrennt. Die wässrige Phase wird mehrfach mit MTBE extrahiert, und die vereinigten organischen Phasen werden mit Wasser und gesättigter Natriumchloridlösung gewaschen. Die Lösung wird mit Natriumsulfat getrocknet und vollständig konzentriert. Das Rohprodukt wird säulenchromatographisch (SiO₂, *n*-Heptan) gereinigt. Die weitere Reinigung erfolgt durch Umkristallisation aus Ethanol und *n*-Heptan. 2-(3,5-Difluor-4'-propyl-biphenyl-4-yl)-5-ethyl-thiophen wird als farbloser Feststoff (Schmp. 45 °C) erhalten.

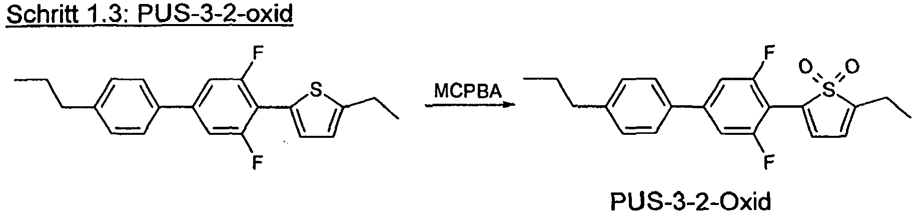

3.2 g (13.0 mmol) MCPBA (5.8 mmol) werden in 20 ml Dichlormethan gelöst und bei T< 25°C mit einer Lösung von 2.0 g (5.8 mmol) 2-(3,5-Difluor-4'-propyl-biphenyl-4-yl)-5-ethyl-thiophen in 5 ml Dichlormethan versetzt. Nach 15 h Bei Raumtemp. wird das Reaktionsgemisch mit Dichlormethan verdünnt und auf ges. Natriumhydrogencarbonatlösung gegeben. Die wässerige Phase wird mit Dichlormethan extrahiert, die vereinigten organischen Phasen mit ges. Natriumchloridlösung gewaschen, über Natriumsulfat getrocknet und eingeengt. Der Rückstand über Kieselgel gegeben (DCM) und aus THF Kristallisiert. Ausbeute: 39 %. Schmp.: 142 °C.

### Beispiel 2:

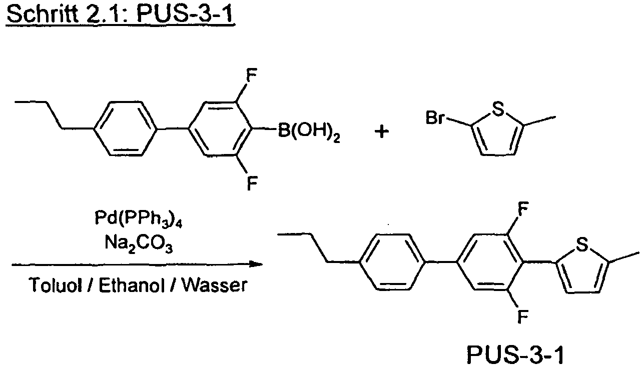

Eine Mischung aus 5,50 g (31,0 mmol) 2-Brom-5-methylthiophen, 9,0 g (32,6 mmol) 4'-Propyl-3,5-difluor-4-biphenylboronsäure, 1,83 g (1,58 mmol) Tetrakis-(triphenylphosphin)palladium(0) und 36 ml 2 N Natriumcarbonatlsg. in 90 ml Toluol/Ethanol (1 : 2) wird 16 h zum Rückfluss erhitzt. Nach dem Abkühlen wird die organische Phase abgetrennt, und die wässrige Phase wird mehrfach mit Toluol extrahiert. Die vereinigten organischen Phasen werden mit Wasser und ges. Natriumchloridlsg. gewaschen. Die Lösung wird mit Natriumsulfat getrocknet und vollständig konzentriert. Der Rückstand wird säulenchromatographisch (SiO₂, *n*-Heptan : Toluol = 9 : 1) gereinigt. Weitere Reinigung erfolgt durch Umkristallisation aus Ethanol und *n-*Heptan; 2-(3,5-Difluor-4'-propyl-biphenyl-4-yl)-5-methyl-thiophen (PUS-3-1) wird als farbloser Feststoff (Schmp. 75 °C) erhalten.

**MS (EI)**: m/z(%) = 328 (100, M⁺), 299 (79, [M - C₂H₅]⁺)
Δε = +3,1
Δn = 0,3055
γ₁ = 102 mPa·s
K 75 N 107 l

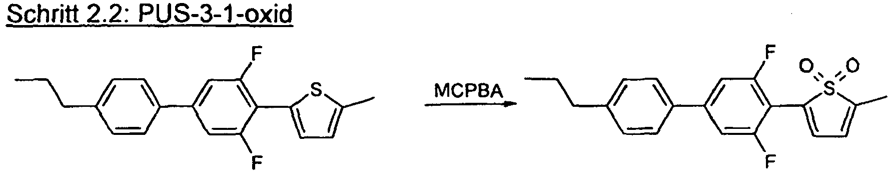

Die Oxidation zum Dioxid erfolgt nach obiger Vorschrift.

### Beispiel 3

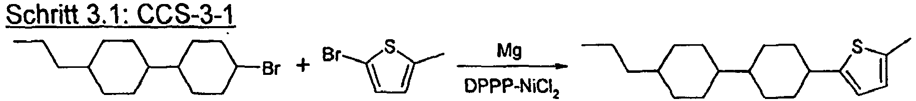

Eine Lösung des aus 60.0 g (209 mmol) des Cyclohexylbromids und 5.0 g (206 mmol) Magnesiumspäne in 700 ml Diethylether hergestellten Grignardreagenzes wird bei 20°C bis 30 °C in eine Lösung von 25.0 g (141 mmol) des Thiophenbromids in 300 ml Diethylether versetzt mit 11.0 g (20.3 mmol) DPPP-NiCl₂ gegeben. Der Ansatz wird 1 h zum Sieden erhitzt. Anschließend wird das Reaktionsgemisch auf mit 2 N Salzsäure angesäuertes Eis/Wassergemisch gegeben. Die wässerige Phase wird mit MTB-Ether extrahiert, die vereinigten organischen Phasen werden mit ges. Natriumchloridlösung gewaschen, über Natriumsulfat getrocknet und eingeengt. Der Rückstand wurde über Kieselgel gegeben (n-Heptan) und aus Aceton kristallisiert. Ausbeute 5 %.

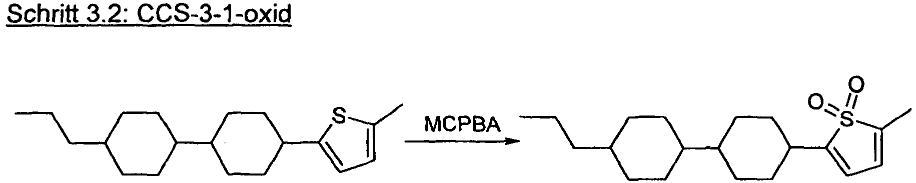

Die Oxidation zum Dioxid erfolgt nach der entsprechenden Vorschrift unter Beispiel 1 (1.3). Ausbeute 30 %. Schmp.: 151 °C.

### Mischungsbeispiele

### Thiophenhaltige Grundmischung M1

Eine nematische FK-Mischung wird wie folgt formuliert

| | | | |
|---|---|---|---|
| PGUQU-3-F | 8,0 % | Kp. | +74°C |
| CPGU-3-OT | 6,5 % | Δn | 0,132 |
| PUQU-3-F | 14,0 % | Δε | +7,2 |
| CC-3-V | 44,0 % | HR₁₀₀ (1 h UV) | 50% |
| CCP-V-1 | 7,5 % | RT (1 h UV) | 6,20 ms |
| PGP-2-2V | 4,0 % | | |
| PUS-3-2 | 16,0 % | | |

### Mischungsbeispiel 1 (mit SS-2-2 stabilisierte Grundmischung M1)

Eine erfindungsgemäße nematische FK-Mischung wird wie folgt formuliert

| | | | |
|---|---|---|---|
| Mischung M1 | 99,0 % | HR₁₀₀ (1 h UV) | 64 % |
| SS-2-2 | 1,0 % | RT (1h UV) | 6,01 ms |

Die stabilisierte Mischung weist nach 1 h UV-Bestrahlung gegenüber der Grundmischung M1 einen höheren VHR-Wert und eine geringere Schaltzeit auf.

### Mischungsbeispiel 2 (mit SS-2-2 + PUS-3-2-Oxid stabilisierte Grundmischung M1)

Eine erfindungsgemäße nematische FK-Mischung wird wie folgt formuliert

| | | | |
|---|---|---|---|
| Mischung M1 | 98,95 % | HR₁₀₀ (1 h UV) | 74 % |
| SS-2-2 | 1,0 % | RT (1 h UV) | 5,90 ms |
| PUS-3-2-Oxid | 0,05 % | | |

Die stabilisierte Mischung weist nach 1 h UV-Bestrahlung gegenüber der Grundmischung M1 einen höheren VHR-Wert und eine geringere Schaltzeit auf.

### Thiophenhaltige Grundmischung M2

Eine nematische FK-Mischung wird wie folgt formuliert:

| | | | |
|---|---|---|---|
| PGUQU-3-F | 11,0 % | Kp. | + 74 °C |
| CPGU-3-OT | 5,0 % | Δn | 0,133 |
| APUQU-3-F | 12,5 % | Δε | +77 |
| CC-3-V | 48, 5 % | HR₁₀₀ (1 h UV) | 54% |
| PP-1-2V1 | 7,0 % | RT (1 h UV) | 5,94 ms |
| PUS-3-2 | 16,0 % | | |

### Mischungsbeispiel 3 (mit SS-2-2 + PUS-3-2-Oxid stabilisierte Grundmischung M2)

Eine erfindungsgemäße nematische FK-Mischung wird wie folgt formuliert

| | | | |
|---|---|---|---|
| Mischung M2 | 98, 95 % | HR₁₀₀ (1 h UV) | 74 % |
| SS-2-2 | 1,0 % | RT (1 h UV) | 5,68 ms |
| P U S-3-2-Oxid | 0,05 % | | |

Die stabilisierte Mischung weist nach 1 h UV-Bestrahlung gegenüber der Grundmischung M2 einen höheren VHR-Wert und eine geringere Schaltzeit auf.

Weitere Kombinationen der Ausführungsformen und Varianten der Erfindung ergeben sich aus den folgenden Ansprüchen.

## Patentansprüche

1. FK-Medium enthaltend eine oder mehrere Verbindungen der Formel I 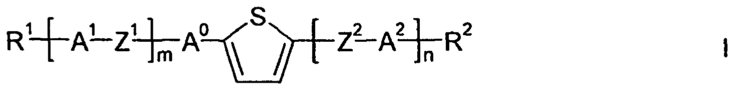 worin die einzelnen Reste folgende Bedeutung besitzen
R¹ und R² H, F, Cl. Br, -CN, -SCN, -NCS, SF₅ oder geradkettiges oder verzweigtes Alkyl mit 1 bis 12 C-Atomen, worin auch eine oder mehrere nicht benachbarte CH₂-Gruppen jeweils unabhängig voneinander durch -CH=CH-, -C≡C-, -O-, -CO-, -CO-O-, -O-CO-, -O-CO-O- so ersetzt sein können, dass O-Atome nicht direkt miteinander verknüpft sind, und worin auch ein oder mehrere H-Atome durch F, Cl oder Br ersetzt sein können,
A⁰, A¹, A² jeweils unabhängig voneinander Phenylen-1,4-diyl, worin auch eine oder zwei CH-Gruppen durch N ersetzt sein können und ein oder mehrere H-Atome durch Halogen, CN, CH₃, CHF₂, CH₂F, OCH₃, OCHF₂ oder OCF₃ ersetzt sein können, Cyclohexan-1,4-diyl, worin auch eine oder zwei nicht benachbarte CH₂ -Gruppen unabhängig voneinander durch 0 und/oder S ersetzt sein können, und ein oder mehrere H-Atome durch F ersetzt sein können, Cyclohexen-1,4-diyl, Bicyclo[1.1.1]pentan-1,3-diyl, Bicyclo[2.2.2]octan-1,4-diyl, Spiro[3.3]heptan-2,6-diyl, Tetrahydropyran-2,5-diyl, oder 1,3-Dioxan-2,5-diyl,
Z¹ und Z² jeweils unabhängig voneinander-CF₂O-, -OCF₂-, -CH₂O-, -OCH₂-, -CO-O-, -O-CO-, -C₂H₄- -C₂F₄-, --CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C=C- oder eine Einfachbindung, und
m und n jeweils unabhängig voneinander 0, 1, 2 oder 3,
**dadurch gekennzeichnet, dass** es zusätzlich eine oder mehre Verbindungen der Formel S1, 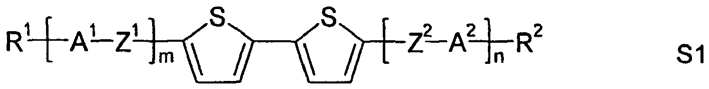 worin die einzelnen Reste folgende Bedeutung besitzen:
R¹ und R² H, F, Cl. Br, -CN, -SCN, -NCS, SF₅ oder geradkettiges oder verzweigtes Alkyl mit 1 bis 12 C-Atomen, worin auch eine oder mehrere nicht benachbarte CH₂-Gruppen jeweils unabhängig voneinander durch -CH=CH-, -C≡C-, -O-, -CO-, -CO-O-, -O-CO-, -O-CO-O- so ersetzt sein können, dass O-Atome nicht direkt miteinander verknüpft sind, und worin auch ein oder mehrere H-Atome durch F, Cl oder Br ersetzt sein können,
A¹ und A² jeweils unabhängig voneinander Phenylen-1,4-diyl, worin auch eine oder zwei CH-Gruppen durch N ersetzt sein können und ein oder mehrere H-Atome durch Halogen, CN, CH₃, CHF₂, CH₂F, OCH₃, OCHF₂ oder OCF₃ ersetzt sein können, Cyclohexan-1,4-diyl, worin auch eine oder zwei nicht benachbarte CH₂ -Gruppen unabhängig voneinander durch O und/oder S ersetzt sein können, und ein oder mehrere H-Atome durch F ersetzt sein können, Cyclohexen-1,4-diyl, Bicyclo[1.1.1]pentan-1,3-diyl, Bicyclo[2.2.2]octan-1,4-diyl, Spiro[3.3]heptan-2,6-diyl, Tetrahydropyran-2,5-diyl, oder 1,3-Dioxan-2,5-diyl,
Z¹ und Z² jeweils unabhängig voneinander-CF₂O-, -OCF₂-, -CH₂O-, -OCH₂-, -CO-O-, -O-CO-, -C₂H₄- -C₂F₄-, -CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- oder eine Einfachbindung, und
m und n jeweils unabhängig voneinander 0, 1, 2 oder 3,
und/oder
eine oder mehrere Verbindungen der Formel S2, 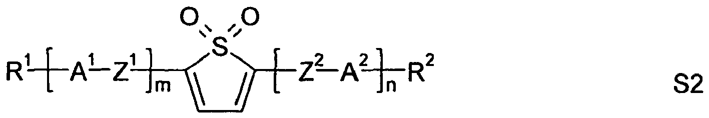 worin die einzelnen Reste folgende Bedeutung besitzen:
R¹ und R² H, F, Cl, Br, -CN, -SCN, -NCS, SF₅ oder geradkettiges oder verzweigtes Alkyl mit 1 bis 12 C-Atomen, worin auch eine oder mehrere nicht benachbarte CH₂-Gruppen jeweils unabhängig voneinander durch -CH=CH-, -C≡C-, -O-, -CO-, -CO-O-, -O-CO-, -O-CO-O- so ersetzt sein können, dass O-Atome nicht direkt miteinander verknüpft sind, und worin auch ein oder mehrere H-Atome durch F, Cl oder Br ersetzt sein können,
A¹ und A² jeweils unabhängig voneinander Phenylen-1,4-diyl, worin auch eine oder zwei CH-Gruppen durch N ersetzt sein können und ein oder mehrere H-Atome durch Halogen, CN, CH₃, CHF₂, CH₂F, OCH₃, OCHF₂ oder OCF₃ ersetzt sein können, Cyclohexan-1,4-diyl, worin auch eine oder zwei nicht benachbarte CH₂ -Gruppen unabhängig voneinander durch O und/oder S ersetzt sein können, und ein oder mehrere H-Atome durch F ersetzt sein können, Cyclohexen-1,4-diyl, Bicyclo[1.1.1]pentan-1,3-diyl, Bicyclo[2.2.2]octan-1,4-diyl, Spiro[3.3]heptan-2,6-diyl, Tetrahydropyran-2,5-diyl, oder 1,3-Dioxan-2,5-diyl,
Z¹ und Z² jeweils unabhängig voneinander -CF₂O-, -OCF₂-, -CH₂O-, -OCH₂-, -CO-O-, -O-CO-, -C₂H₄- -C₂F₄-, -CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- oder eine Einfachbindung, und
m und n jeweils unabhängig voneinander 0, 1, 2 oder 3,
enthält.

2. FK-Medium nach Anspruch 1, **dadurch gekennzeichnet, dass** es eine oder mehrere Verbindungen der Formel S1 enthält.

3. FK-Medium nach Anspruch 1 oder 2, **dadurch gekennzeichnet, dass** es eine oder mehrere Verbindungen der Formel S1 und eine oder mehrere Verbindungen der Formel S2 enthält.

4. FK-Medium nach einem oder mehreren der Ansprüche 1 bis 3, **dadurch gekennzeichnet, dass** in Formel I n 0, m 0 oder 1, und Z¹ eine Einfachbindung bedeuten.

5. FK-Medium nach einem oder mehreren der Ansprüche 1 bis 4, **dadurch gekennzeichnet, dass** es zusätzliche eine oder mehrere Verbindungen der Formel II und/oder III enthält: 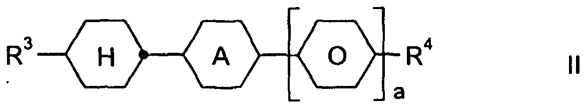 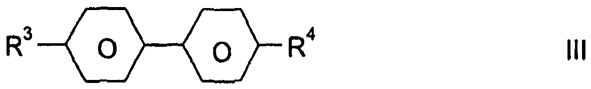 worin
A 1,4-Phenylen oder trans-1,4-Cyclohexylen bedeutet,
a 0 oder 1 ist,
R³ Alkyl oder Alkenyl mit bis zu 9 C-Atomen bedeutet, und
R⁴ Alkyl mit 1 bis 12 C-Atomen, wobei auch eine oder zwei nicht benachbarte CH₂-Gruppen durch -O-, -CH=CH-, -CO-, -OCO-oder -COO- so ersetzt sein können, dass O-Atome nicht direkt miteinander verknüpft sind, und vorzugsweise Alkyl mit 1 bis 12 C-Atomen oder Alkenyl mit 2 bis 9 C-Atomen bedeutet.

6. FK-Medium nach einem oder mehreren der Ansprüche 1 bis 5, **dadurch gekennzeichnet, dass** es zusätzlich eine oder mehrere Verbindungen ausgewählt aus der Gruppe bestehend aus folgenden Formeln enthält 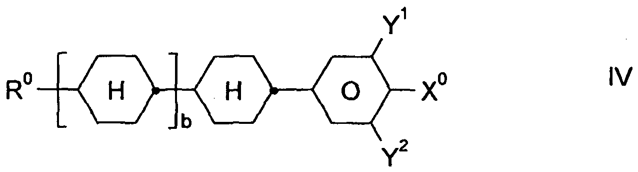 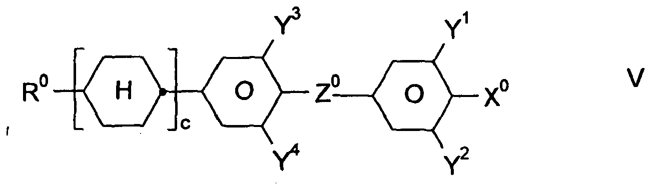 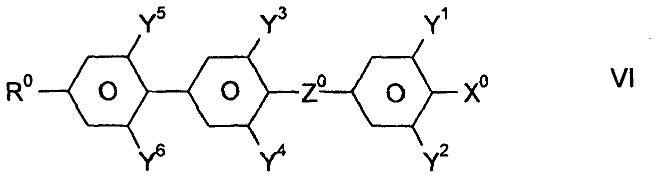 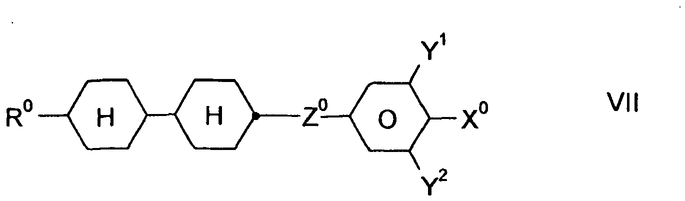 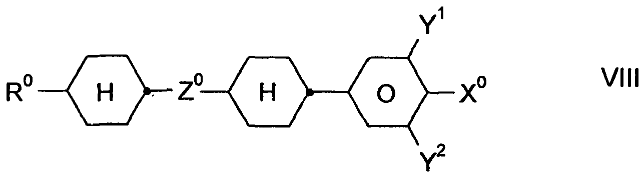 worin
R⁰ einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 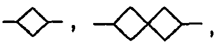 -O-, -CO-O- oder -O-CO- so ersetzt sein können, dass O-Atome nicht direkt miteinander verknüpft sind, und worin auch ein oder mehrere H-Atome durch Halogen ersetzt sein können,
X⁰ F, Cl, CN, SF₅, SCN, NCS, einen halogenierten Alkylrest, halogenierten Alkenylrest, halogenierten Alkoxyrest oder halogenierten Alkenyloxyrest mit jeweils bis zu 6 C-Atomen,
Y¹⁻⁶ jeweils unabhängig voneinander H oder F,
Z⁰ -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- oder -OCF₂-, in den Formeln V und VI auch eine Einfachbindung, und
b und c jeweils unabhängig voneinander 0 oder 1
bedeuten.

7. Verbindungen der Formel S2 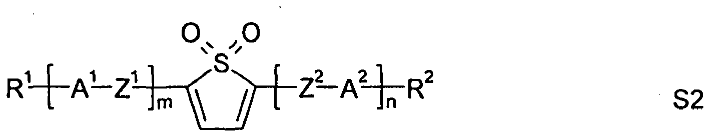 worin die einzelnen Reste wie im Anspruch 1 definiert sind.

8. Verbindungen nach Anspruch 7, **dadurch gekennzeichnet, dass**
m 1, 2 oder 3, und
n 0 bedeutet.

9. Verbindungen nach Anspruch 7 oder 8, **dadurch gekennzeichnet, dass** Z¹ und Z² Einfachbindungen sind.

10. Verbindungen nach einem oder mehreren der Ansprüche 7 bis 9, **dadurch gekennzeichnet, dass** R¹ und R² nicht H bedeuten.

11. Verwendung von Verbindungen der Formel S1 und/oder S2 nach Anspruch 1 zur Stabilisierung eines FK-Mediums enthaltend eine oder mehrere Thiophenverbindungen der Formel I nach Anspruch 1 oder 4.

12. FK-Medium enthaltend eine oder mehrere Verbindungen der Formel S2.

13. Verfahren zur Herstellung von Verbindungen der Formel S2 gemäß einem oder mehreren der Ansprüche 7 bis 10, **dadurch gekennzeichnet, dass** man eine Thiophenverbindung an der Thiopheneinheit zu dem Thiophenoxid der Formel S2 oxidiert.

14. Verwendung eines FK-Mediums nach einem oder mehreren der Ansprüche 1 bis 6 für eine elektrooptische Vorrichtung.

15. FK-Anzeige enthaltend ein FK-Medium nach einem oder mehreren der Ansprüche 1 bis 6 oder eine oder mehrere Verbindungen der Formel S2 nach einem oder mehreren der Ansprüche 7 bis 10.

## Claims

1. LC medium comprising one or more compounds of the formula I 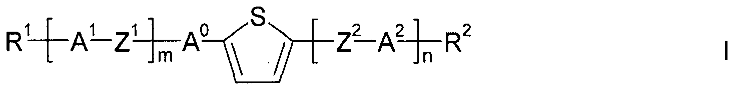 in which the individual radicals have the following meanings:
R¹ and R² denote H, F, Cl, Br, -CN, -SCN, -NCS, SF₅ or straight-chain or branched alkyl having 1 to 12 C atoms, in which, in addition, one or more non-adjacent CH₂ groups may each be replaced, independently of one another, by -CH=CH-, -C≡C-, -O-, -CO-, -CO-O-, -O-CO-, -O-CO-O-in such a way that O atoms are not linked directly to one another, and in which, in addition, one or more H atoms may be replaced by F, Cl or Br,
A⁰, A¹, A² each, independently of one another, denote phenylene-1,4-diyl, in which, in addition, one or two CH groups may be replaced by N and one or more H atoms may be replaced by halogen, CN, CH₃, CHF₂, CH₂F, OCH₃, OCHF₂ or OCF₃, cyclohexane-1,4-diyl, in which, in addition, one or two non-adjacent CH₂ groups may be replaced, independently of one another, by O and/or S and one or more H atoms may be replaced by F, cyclohexene-1,4-diyl, bicyclo[1.1.1]pentane-1,3-diyl, bicyclo[2.2.2]octane-1,4-diyl, spiro[3.3]heptane-2,6-diyl, tetrahydropyran-2,5-diyl or 1,3-dioxane-2,5-diyl,
Z¹ and Z² each, independently of one another, denote -CF₂O-, -OCF₂-, -CH₂O-, -OCH₂-, -CO-O-, -O-CO-, -C₂H₄-, -C₂F₄-, -CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- or a single bond, and
m and n each, independently of one another, denote 0, 1, 2 or 3,
**characterised in that** it additionally comprises one or more compounds of the formula S1, 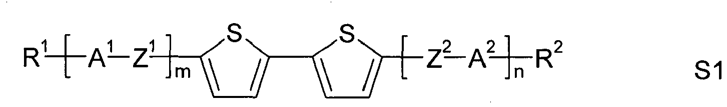 in which the individual radicals have the following meanings:
R¹ and R² denote H, F, Cl, Br, -CN, -SCN, -NCS, SF₅ or straight-chain or branched alkyl having 1 to 12 C atoms, in which, in addition, one or more non-adjacent CH₂ groups may each be replaced, independently of one another, by -CH=CH-, -C≡C-, -O-, -CO-, -CO-O-, -O-CO-, -O-CO-O- in such a way that O atoms are not linked directly to one another, and in which, in addition, one or more H atoms may be replaced by F, Cl or Br,
A¹ and A² each, independently of one another, denote phenylene-1,4-diyl, in which, in addition, one or two CH groups may be replaced by N and one or more H atoms may be replaced by halogen, CN, CH₃, CHF₂, CH₂F, OCH₃, OCHF₂ or OCF₃, cyclohexane-1,4-diyl, in which, in addition, one or two non-adjacent CH₂ groups may be replaced, independently of one another, by O and/or S and one or more H atoms may be replaced by F, cyclohexene-1,4-diyl, bicyclo[1.1.1]pentane-1,3-diyl, bicyclo[2.2.2]octane-1,4-diyl, spiro[3.3]heptane-2,6-diyl, tetrahydropyran-2,5-diyl or 1,3-dioxane-2,5-diyl,
Z¹ and Z² each, independently of one another, denote -CF₂O-, -OCF₂-, -CH₂O-, -OCH₂-, -CO-O-, -O-CO-, -C₂H₄-, -C₂F₄-, -CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- or a single bond, and
m and n each, independently of one another, denote 0, 1, 2 or 3,
and/or
one or more compounds of the formula S2, 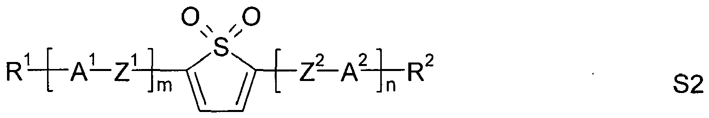 in which the individual radicals have the following meanings:
R¹ and R² denote H, F, Cl, Br, -CN, -SCN, -NCS, SF₅ or straight-chain or branched alkyl having 1 to 12 C atoms, in which, in addition, one or more non-adjacent CH₂ groups may each be replaced, independently of one another, by -CH=CH-, -C≡C-, -O-, -CO-, -CO-O-, -O-CO-, -O-CO-O-in such a way that O atoms are not linked directly to one another, and in which, in addition, one or more H atoms may be replaced by F, Cl or Br,
A¹ and A² each, independently of one another, denote phenylene-1,4-diyl, in which, in addition, one or two CH groups may be replaced by N and one or more H atoms may be replaced by halogen, CN, CH₃, CHF₂, CH₂F, OCH₃, OCHF₂ or OCF₃, cyclohexane-1,4-diyl, in which, in addition, one or two non-adjacent CH₂ groups may be replaced, independently of one another, by O and/or S and one or more H atoms may be replaced by F, cyclohexene-1,4-diyl, bicyclo[1.1.1]pentane-1,3-diyl, bicyclo[2.2.2]octane-1,4-diyl, spiro[3.3]heptane-2,6-diyl, tetrahydropyran-2,5-diyl or 1,3-dioxane-2,5-diyl,
Z¹ and Z² each, independently of one another, denote -CF₂O-, -OCF₂-, -CH₂O-, -OCH₂-, -CO-O-, -O-CO-, -C₂H₄-, -C₂F₄-, -CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- or a single bond, and
m and n each, independently of one another, denote 0, 1, 2 or 3.

2. LC medium according to Claim 1, **characterised in that** it comprises one or more compounds of the formula S1.

3. LC medium according to Claim 1 or 2, **characterised in that** it comprises one or more compounds of the formula S1 and one or more compounds of the formula S2.

4. LC medium according to one or more of Claims 1 to 3, **characterised in that**, in formula I, n denotes 0, m denotes 0 or 1, and Z¹ denotes a single bond.

5. LC medium according to one or more of Claims 1 to 4, **characterised in that** it additionally comprises one or more compounds of the formulae II and/or III: 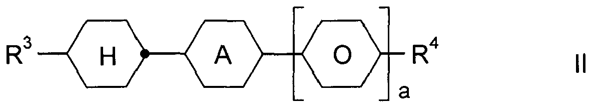 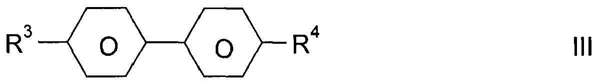 in which
A denotes 1,4-phenylene or trans-1,4-cyclohexylene,
a is 0 or 1,
R³ denotes alkyl or alkenyl having up to 9 C atoms, and
R⁴ denotes alkyl having 1 to 12 C atoms, where, in addition, one or two non-adjacent CH₂ groups may be replaced by -O-, -CH=CH-, -CO-, -OCO- or -COO- in such a way that O atoms are not linked directly to one another, and preferably denotes alkyl having 1 to 12 C atoms or alkenyl having 2 to 9 C atoms.

6. LC medium according to one or more of Claims 1 to 5, **characterised in that** it additionally comprises one or more compounds selected from the group consisting of the following formulae: 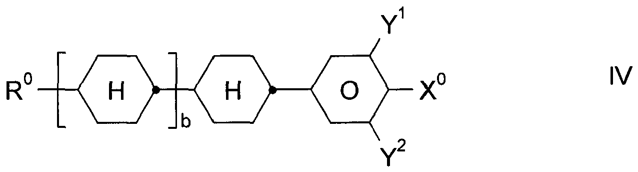 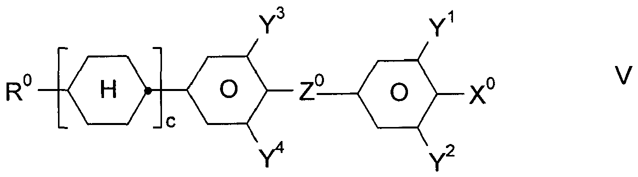 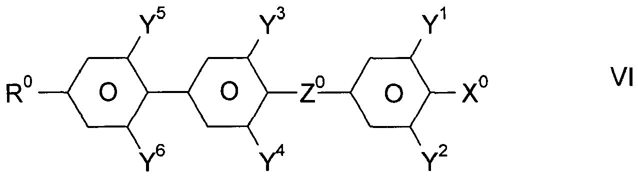 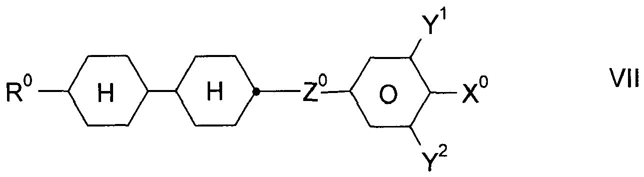 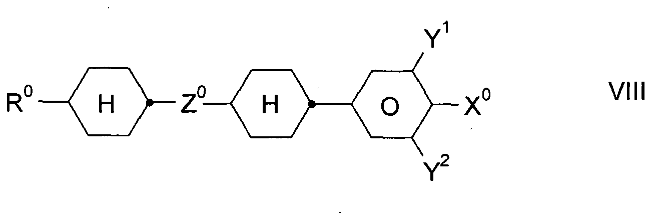 in which
R⁰ denotes an alkyl or alkoxy radical having 1 to 15 C atoms, where, in addition, one or more CH₂ groups in these radicals may each, be replaced independently of one another, by -C≡C-, -CF₂O-, -CH=CH-, 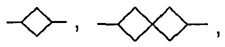 -O-, -CO-O- or -O-CO- in such a way that O atoms are not linked directly to one another, and in which, in addition, one or more H atoms may be replaced by halogen,
X⁰ denotes F, Cl, CN, SF₅, SCN, NCS, a halogenated alkyl radical, halogenated alkenyl radical, halogenated alkoxy radical or halogenated alkenyloxy radical, each having up to 6 C atoms,
Y¹⁻⁶ each, independently of one another, denote H or F,
Z⁰ denotes -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- or -OCF₂-, in the formulae V and VI also a single bond, and
b and c each, independently of one another, denote 0 or 1.

7. Compounds of the formula S2 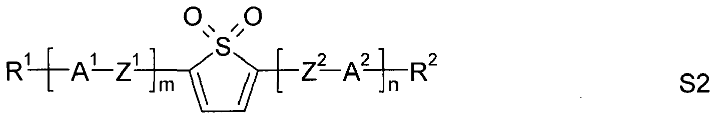 in which the individual radicals are as defined in Claim 1.

8. Compounds according to Claim 7, **characterised in that**
m denotes 1, 2 or 3, and
n denotes 0.

9. Compounds according to Claim 7 or 8, **characterised in that** Z¹ and Z² are single bonds.

10. Compounds according to one or more of Claims 7 to 9, **characterised in that** R¹ and R² do not denote H.

11. Use of compounds of the formulae S1 and/or S2 according to Claim 1 for the stabilisation of an LC medium comprising one or more thiophene compounds of the formula I according to Claim 1 or 4.

12. LC medium comprising one or more compounds of the formula S2.

13. Process for the preparation of compounds of the formula S2 according to one or more of Claims 7 to 10, **characterised in that** a thiophene compound on the thiophene unit is oxidised to the thiophene oxide of the formula S2.

14. Use of an LC medium according to one or more of Claims 1 to 6 for an electro-optical device.

15. LC display containing an LC medium according to one or more of Claims 1 to 6 or one or more compounds of the formula S2 according to one or more of Claims 7 to 10.

## Revendications

1. Milieu LC comprenant un ou plusieurs composé(s) de la formule I : 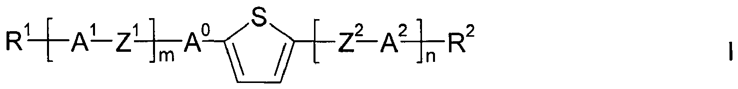 dans laquelle les radicaux individuels présentent les significations qui suivent :
R¹ et R² représentent H, F, Cl, Br, -CN, -SCN, -NCS, SF₅ ou alkyle en chaîne droite ou ramifié comportant de 1 à 12 atomes de C, où, en outre, un ou plusieurs groupe(s) CH₂ non adjacents peut/peuvent chacun être remplacé(s), indépendamment les uns des autres, par -CH=CH-, -C≡C-, -O-, -CO-, -CO-O-, -O-CO-, -O-CO-Ode telle sorte que des atomes de O ne soient pas liés directement les uns aux autres et où, en outre, un ou plusieurs atome(s) de H peut/peuvent être remplacé(s) par F, Cl ou Br,
A⁰, A¹, A² représentent, chacun indépendamment des autres, phénylène-1,4-diyle, où, en outre, un ou deux groupe(s) CH peut/peuvent être remplacé(s) par N et un ou plusieurs atome(s) de H peut/peuvent être remplacé(s) par halogène, CN, CH₃, CHF₂, CH₂F, OCH₃, OCHF₂ ou OCF₃, cyclohexane-1,4-diyle, où, en outre, un ou deux groupe(s) CH₂ non adjacents peut/peuvent être remplacé(s), indépendamment l'un de l'autre, par O et/ou S et un ou plusieurs atome(s) de H peut/ peuvent être remplacé(s) par F, cyclohexène-1,4-diyle, bicyclo[1.1.1]pentane-1,3-diyle, bicyclo[2.2.2]octane-1,4-diyle, spiro[3.3]heptane-2,6-diyle, tétrahydropyran-2,5-diyle ou 1,3-dioxane-2,5-diyle,
Z¹ et Z² représentent, chacun indépendamment de l'autre, -CF₂O-, -OCF₂-, -CH₂O-, -OCH₂-, -CO-O-, -O-CO-, -C₂H₄-, -C₂F₄-, -CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- ou une liaison simple, et
m et n représentent, chacun indépendamment de l'autre, 0, 1, 2 ou 3,
**caractérisé en ce qu'**il comprend de façon additionnelle un ou plusieurs composé(s) de la formule S1 : 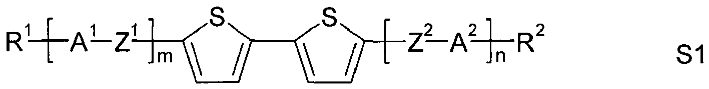 dans laquelle les radicaux individuels présentent les significations qui suivent :
R¹ et R² représentent H, F, Cl, Br, -CN, -SCN, -NCS, SF₅ ou alkyle en chaîne droite ou ramifié comportant de 1 à 12 atomes de C, où, en outre, un ou plusieurs groupe(s) CH₂ non adjacent(s) peut/peuvent chacun être remplacé(s), indépendamment les uns des autres, par -CH=CH-, -C≡C-, -O-, -CO-, -CO-O-, -O-CO-, -O-CO-Ode telle sorte que des atomes de O ne soient pas liés directement les uns aux autres et où, en outre, un ou plusieurs atome(s) de H peut/peuvent être remplacé(s) par F, Cl ou Br,
A¹ et A² représentent, chacun indépendamment de l'autre, phénylène-1,4-diyle, où, en outre, un ou deux groupes CH peut/peuvent être remplacé(s) par N et un ou plusieurs atome(s) de H peut/peuvent être remplacé(s) par halogène, CN, CH₃, CHF₂, CH₂F, OCH₃, OCHF₂ ou OCF₃, cyclohexane-1,4-diyle, où, en outre, un ou deux groupe(s) CH₂ non adjacents peut/peuvent être remplacé(s), indépendamment l'un de l'autre, par O et/ou S et un ou plusieurs atome(s) de H peut/peuvent être remplacé(s) par F, cyclohexène-1,4-diyle, bicyclo-[1.1.1]pentane-1,3-diyle, bicyclo[2.2.2]octane-1,4-diyle, spiro[3.3]heptane-2,6-diyle, tétrahydropyran-2,5-diyle ou 1,3-dioxane-2,5-diyle,
Z¹ et Z² représentent, chacun indépendamment de l'autre, -CF₂O-, -OCF₂-, -CH₂O-, -OCH₂-, -CO-O-, -O-CO-, -C₂H₄-, -C₂F₄-, -CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- ou une liaison simple, et
m et n représentent, chacun indépendamment de l'autre, 0, 1, 2 ou 3,
et/ou
un ou plusieurs composé(s) de la formule S2 : 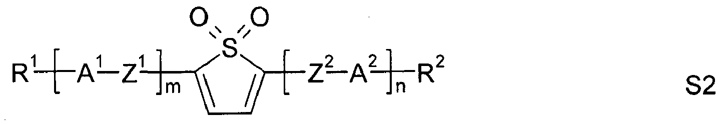 dans laquelle les radicaux individuels présentent les significations qui suivent :
R¹ et R² représentent H, F, Cl, Br, -CN, -SCN, -NCS, SF₅ ou alkyle en chaîne droite ou ramifié comportant de 1 à 12 atomes de C, où, en outre, un ou plusieurs groupe(s) CH₂ non adjacents peut/peuvent chacun être remplacé(s), indépendamment les uns des autres, par -CH=CH-, -C≡C-, -O-, -CO-, -CO-O-, -O-CO-, -O-CO-Ode telle sorte que des atomes de O ne soient pas liés directement les uns aux autres et où, en outre, un ou plusieurs atome(s) de H peut/peuvent être remplacé(s) par F, Cl ou Br,
A¹ et A² représentent, chacun indépendamment de l'autre, phénylène-1,4-diyle, où, en outre, un ou deux groupe(s) CH peut/peuvent être remplacé(s) par N et un ou plusieurs atome(s) de H peut/peuvent être remplacé(s) par halogène, CN, CH₃, CHF₂, CH₂F, OCH₃, OCHF₂ ou OCF₃, cyclohexane-1,4-diyle, où, en outre, un ou deux groupe(s) CH₂ non adjacents peut/peuvent être remplacé(s), indépendamment l'un de l'autre, par O et/ou S et un ou plusieurs atome(s) de H peut/ peuvent être remplacé(s) par F, cyclohexène-1,4-diyle, bicyclo[1.1.1]pentane-1,3-diyle, bicyclo[2.2.2]octane-1,4-diyle, spiro[3.3]heptane-2,6-diyle, tétrahydropyran-2,5-diyle ou 1,3-dioxane-2,5-diyle,
Z¹ et Z² représentent, chacun indépendamment de l'autre, -CF₂O-, -OCF₂-, -CH₂O-, -OCH₂-, -CO-O-, -O-CO-, -C₂H₄-, -C₂F₄-, -CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- ou une liaison simple, et
m et n représentent, chacun indépendamment de l'autre, 0, 1, 2 ou 3.

2. Milieu LC selon la revendication 1, **caractérisé en ce qu'**il comprend un ou plusieurs composé(s) de la formule S1.

3. Milieu LC selon la revendication 1 ou 2, **caractérisé en ce qu'**il comprend un ou plusieurs composé(s) de la formule S1 et un ou plusieurs composé(s) de la formule S2.

4. Milieu LC selon une ou plusieurs des revendications 1 à 3, **caractérisé en ce que**, dans la formule I, n représente 0, m représente 0 ou 1, et Z¹ représente une liaison simple.

5. Milieu LC selon une ou plusieurs des revendications 1 à 4, **caractérisé en ce qu'**il comprend de façon additionnelle un ou plusieurs composé(s) des formules II et/ou III : 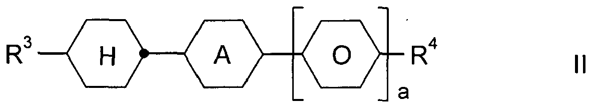 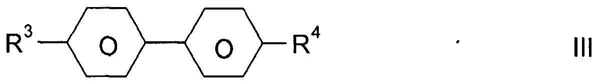 dans lesquelles :
A représente 1,4-phénylène ou trans-1,4-cyclohexylène,
a est 0 ou 1,
R³ représente alkyle ou alkényle comportant jusqu'à 9 atomes de C, et
R⁴ représente alkyle comportant de 1 à 12 atomes de C, où, en outre, un ou deux groupe(s) CH₂ non adjacents peut/peuvent être remplacé(s) par -O-, -CH=CH-, -CO-, -OCO- ou -COO- de telle sorte que des atomes de O ne soient pas liés directement les uns aux autres et de façon préférable, représente alkyle comportant de 1 à 12 atomes de C ou alkényle comportant de 2 à 9 atomes de C.

6. Milieu LC selon une ou plusieurs des revendications 1 à 5, **caractérisé en ce qu'**il comprend de façon additionnelle un ou plusieurs composé(s) choisi(s) parmi le groupe constitué par les formules qui suivent : 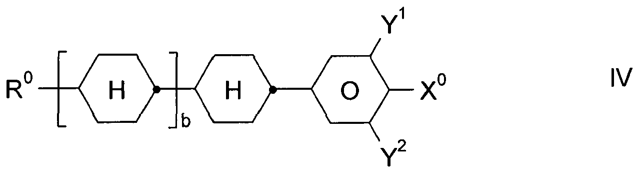 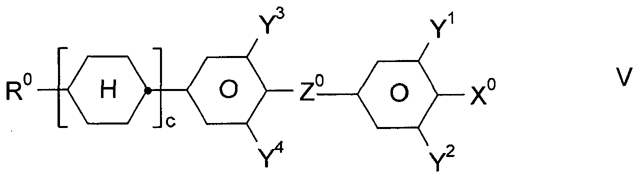 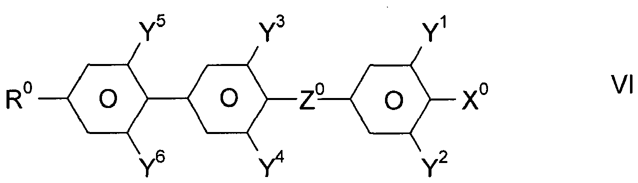 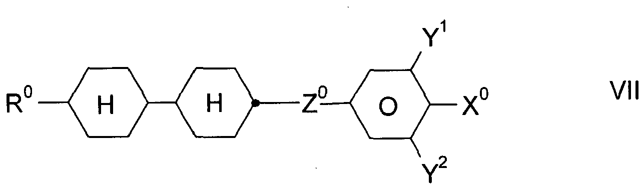 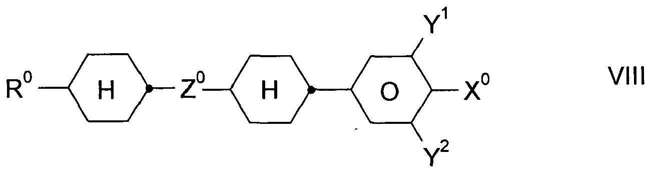 dans lesquelles :
R⁰ représente un radical alkyle ou alcoxy comportant de 1 à 15 atomes de C, où, en outre, un ou plusieurs groupe(s) CH₂ dans ces radicaux peut/peuvent chacun être remplacé(s), indépendamment les uns des autres, par -C≡C-, -CF₂O-, -CH=CH-, 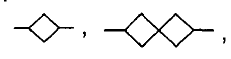 -O-, -CO-O- ou -O-CO- de telle sorte que des atomes de O ne soient pas liés directement les uns aux autres et où, en outre, un ou plusieurs atome(s) de H peut/ peuvent être remplacé(s) par halogène,
X⁰ représente F, Cl, CN, SF₅, SCN, NCS, un radical alkyle halogéné, un radical alkényle halogéné, un radical alcoxy halogéné ou un radical alkényloxy halogéné, chacun comportant jusqu'à 6 atomes de C,
Y¹⁻⁶ représentent, chacun indépendamment des autres, H ou F,
Z⁰ représente -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- ou -OCF₂-, dans les formules V et VI également une liaison simple, et
b et c représentent, chacun indépendamment de l'autre, 0 ou 1.

7. Composés de la formule S2 : 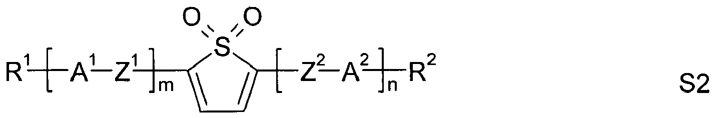 dans laquelle les radicaux individuels sont comme défini selon la revendication 1.

8. Composés selon la revendication 7, **caractérisés en ce que** :
m représente 1, 2 ou 3, et
n représente 0.

9. Composés selon la revendication 7 ou 8, **caractérisés en ce que** Z¹ et Z² sont des liaisons simples.

10. Composés selon une ou plusieurs des revendications 7 à 9, **caractérisés en ce que** R¹ et R² ne représentent pas H.

11. Utilisation de composés des formules S1 et/ou S2 selon la revendication 1 pour la stabilisation d'un milieu LC comprenant un ou plusieurs composés thiophène de la formule I selon la revendication 1 ou 4.

12. Milieu LC comprenant un ou plusieurs composé(s) de la formule S2.

13. Procédé pour la préparation de composés de la formule S2 selon une ou plusieurs des revendications 7 à 10, **caractérisé en ce qu'**un composé thiophène sur l'unité thiophène est oxydé selon l'oxyde de thiophène de la formule S2.

14. Utilisation d'un milieu LC selon une ou plusieurs des revendications 1 à 6 pour un dispositif électro-optique.

15. Affichage LC contenant un milieu LC selon une ou plusieurs des revendications 1 à 6 ou un ou plusieurs composé(s) de la formule S2 selon une ou plusieurs des revendications 7 à 10.
